# EUROPEAN PATENT APPLICATION

(11) **EP 4 813 400 A1**
(43) Date of publication of application: **30.09.2026**
(21) Application number: 26168701.6
(22) Date of filing: 27.03.2026
(51) Int. Cl.: A61K 35/744, A61K 35/745, A23L 33/135, A61K 31/593, A61K 35/747, A61P 1/00, C12N 5/00

(54) **MISXTURE OF PROBIOTIC BACTERIAL STRAINS IN COMBINATION WITH VITAMIN D, FOR USE IN TREATING IRRITABLE BOWEL SYNDROME**

(30) Priority: 28.03.2025 IT 202500006582
(71) Applicant: Beingpharma S.r.l, 20123 Milano (MI) (IT)
(72) Inventor: COLOMBO, Stefano, 20123 Milano (IT); TATULLI, Leandro, 20123 Milano (IT); MEGNA, Francesca, 20123 Milano (IT)
(74) Representative: Marben S.r.l.

(57) **Abstract**

The present invention relates to a mixture comprising or, alternatively, consisting of eight specific strains of bacteria and vitamin D.

Furthermore, the present invention relates to a composition comprising said mixture and to the use thereof in therapy, preferably in a method of treatment, preventive or curative, of irritable bowel syndrome and/or disorders associated therewith, in IBS patients without constipation.

## Description

The present invention relates to a mixture comprising or, alternatively, consisting of eight specific strains of bacteria and vitamin D.

Furthermore, the present invention relates to a composition comprising said mixture and the use thereof in therapy, preferably in a method of treatment, preventive or curative, of irritable bowel syndrome and/or disorders associated therewith, in subjects with IBS without constipation.

### BACKGROUND OF THE INVENTION

Irritable bowel syndrome (IBS) is a common gastrointestinal functional disorder affecting 15% of the adult population in Italy and is more frequent in females, particularly under 50 years of age.

Irritable bowel syndrome is a disorder of the brain-gut axis that typically manifests itself with abdominal pain associated with a change in the frequency and/or consistency of stools (diarrhoea or constipation), meteorism and the presence of intestinal gas.

Various factors may contribute to the onset of irritable bowel syndrome, including genetic factors, mental stress, a high-fat diet and alcohol.

Furthermore, an alteration of intestinal permeability, in particular an increased intestinal permeability, and low-grade inflammation seem to be relevant in the pathogenesis of irritable bowel syndrome.

The diagnosis of irritable bowel syndrome is generally based on the symptoms, but investigations are conducted to rule out other disorders.

According to ROME IV criteria, irritable bowel syndrome manifests itself in four main forms, each with distinctive characteristics:
1. Form-D (Diarrhoea Predominant)
   - Frequent bowel movements
   - Soft or liquid stools
   - Often associated with urgency
   - Represents about 25% of cases
2. Form-C (Constipation Predominant)
   - Infrequent bowel movements
   - Hard or lumpy stools
   - Feeling of incomplete evacuation
   - Represents about 25% of cases
3. Form-M (Mixed)
   - Alternation of periods with diarrhoea and constipation
   - Variability in symptoms
   - Can be particularly frustrating to manage
   - Represents about 25% of cases
4. Form-U (Unclassified)
   - Bowel irregularity not falling into the other categories
   - Variable symptoms
   - Represents about 25% of cases.

For a diagnosis of irritable bowel syndrome, recurrent abdominal pain that satisfies all these criteria must be present: it has occurred on average at least 1 day a week in the last 3 months; it is associated with two or more of the following characteristics: it is modified by defecation; it is linked to changes in the frequency of bowel movements; and it is linked to changes in the form (appearance) of stools. The symptoms must have started at least 6 months before the diagnosis and have been present in the last 3 months.

Changes in diet can usually alleviate specific symptoms but, above all in more severe cases, it is necessary to have recourse to drugs, such as anticholinergic drugs, antidiarrhoeic drugs, or laxatives. Such drugs are not devoid of side effects. For example, anticholinergic drugs provoke side effects such as dry mouth, blurry vision or urinary hesitancy.

However, even drugs are often ineffective.

The pathogenesis of IBS is still not wholly clear; however, there is increasing evidence to suggest that dysbiosis and intestinal barrier dysfunction might play an important role.

Therefore, the possibility of improving intestinal barrier function by acting on gut microbiota (GM) could be a potential therapeutic weapon in IBS. GM modulation could be achieved in various ways, using diet, prebiotics, or antibiotics, but probiotics represent one of the most interesting, based on their potential pleiotropic effects and their long-term safety. The evidence supporting the effectiveness of probiotics in irritable bowel syndrome is still controversial, even though every probiotic strain could have a theoretically positive effect on intestinal barrier function by improving the mucus layer, increasing the expression of tight junctions, interacting with host microbes and regulating immune cells in the lamina propria. However, not all probiotic strains have shown all these effects at the same time, since some mechanisms are considered species-specific and even strain-specific.

The scientific publication by COCEAN ANA-MARIA ET AL.: "Exploring the gut-brain Axis: Potential therapeutic impact of Psychobiotics on mental health", PROGRESS IN NEURO-PSYCHOPHARMACOLOGY & BIOLOGICAL PSYCHIATRY, ELSEVIER, GB, vol. 134, 22 June 2024 (2024-06-22), XP087568805, ISSN: 0278-5846, DOI: 10.1016/J.PNPBP.2024.111073 shows a mixture of probiotic strains for use in the treatment of neuropsychological disorders.

The scientific publication by FRANCESCA SANTA ET AL.: "Effect of a multi-strain probiotic mixture consumption on anxiety and depression symptoms induced in adult mice by postnatal maternal separation", MICROBIOME, BIOMED CENTRAL LTD, LONDON, UK, vol. 12, no. 1, 19 February 2024 (2024-02-19), pages 1-19, XP021332448, DOI: 10.1186/S40168-024-01752-W shows a mixture of probiotic strains for use in the treatment of alterations in the emotional sphere.

The scientific publication by PETRELLA CARLA ET AL.: "Proneurogenic and neuroprotective effect of a multi strain probiotic mixture in a mouse model of acute inflammation: Involvement of the gut-brain axis", PHARMACOLOGICAL RESEARCH, ELSEVIER.

The scientific publication by AMSTERDAM, NL, vol. 172, 31 July 2021 (2021-07-31), XP086781671, ISSN: 1043-6618, DOI: 10.1016/J.PHRS.2021.105795 shows the positive effects that a mixture of probiotic strains can have in intestinal permeability and inflammation.

In the most severe cases, the patient must also have recourse to non-pharmacological interventions, such as psychotherapy, constant physical activity, adequate sleep at night and a balanced diet.

However, the drugs available to date and the non-pharmacological interventions it is often necessary to adopt present various disadvantages, in terms of effectiveness and safety of use, in the short and long terms.

It is thus necessary to be able to have compositions for the treatment of IBS which are capable of overcoming the disadvantages of the prior art.

In particular, it is useful to provide compositions for the treatment of subjects with IBS but no constipation (briefly, "IBS patients without constipation").

It is further necessary to be able to have compositions for the treatment of IBS and/or disorders associated therewith which are free of side effects and may also be used by frail subjects, such as the elderly, disabled or immunocompromised.

Furthermore, in particular there is a felt need to be able to have products (mixtures and/or compositions) which act on the composition and function of gut microbiota, thereby reducing dysbiosis and promoting gut health.

These aims and still others, which will emerge clearly from the detailed description that follows, are achieved by a mixture comprising or, alternatively, consisting of eight specific strains of bacteria and vitamin D and by the composition comprising said mixture (mixture and/or composition of the invention), which have the technical features claimed in the appended claims.

### SUMMARY OF THE INVENTION

The technical problem that the present invention addresses and solves is to provide a mixture of natural active substances/ingredients, and nutraceutical and/or pharmaceutical compositions comprising them capable of acting upon the composition and function of the gut microbiota, thereby reducing dysbiosis and promoting gut health.

In the context of the present invention, the term "dysbiosis" or "intestinal dysbiosis" is meant to indicate a condition characterised by an alteration in the natural balance of the gut microbiota, the set of microorganisms, mainly bacteria, inside the intestine. In particular, this alteration is largely due to an excessive growth of "bad" bacteria inside the intestine, which provoke the irritation thereof.

The Applicant, following an intense research activity, has developed and fine-tuned a mixture comprising or, alternatively, consisting of specific strains of bacteria and, preferably, vitamin D, which is capable of reducing intestinal permeability, thereby assuring an effective treatment of irritable bowel syndrome and/or disorders associated therewith. A first aspect of the present invention relates to a mixture comprising or, alternatively, consisting of:
i) a bacterial strain belonging to the species *Bifidobacterium animalis* subsp. *lactis,* wherein said strain is the bacterial strain *Bifidobacterium animalis* subsp. *lactis* BL03 deposited by Beingpharma S.r.l. at the BCCM/LMG/LMG on 06.03.2025 with the deposit number LMG P-34006 and a bacterial strain belonging to the species *Bifidobacterium animalis* subsp. *lactis,* wherein said strain is the bacterial strain *Bifidobacterium animalis* subsp. *lactis* BI04 deposited by Beingpharma S.r.l. at the BCCM/LMG/LMG on 06.03.2025 with the deposit number LMG P-34007; and
ii) at least one bacterial strain belonging to the species *Streptococcus thermophilus,* wherein said strain is the bacterial strain *Streptococcus thermophilus* BT01 deposited by Beingpharma S.r.l. at the BCCM/LMG/LMG on 06.03.2025 with the deposit number LMG P-34002; and
iii) at least one bacterial strain belonging to the species *Lactiplantibacillus plantarum,* wherein said strain is the bacterial strain *Lactiplantibacillus plantarum* BP06 deposited by Beingpharma S.r.l. at the BCCM/LMG/LMG on 06.03.2025 with the deposit number LMG P-34005; and
iv) at least one bacterial strain belonging to the species *Lactobacillus acidophilus,* wherein said strain is the bacterial strain species *Lactobacillus acidophilus* BA05 deposited by Beingpharma S.r.l. at the BCCM/LMG/LMG on 06.03.2025 with the deposit number LMG P-34008; and
v) at least one bacterial strain belonging to the species *Lacticaseibacillus paracasei,* wherein said strain is the bacterial strain *Lacticaseibacillus paracasei* BP07 deposited by Beingpharma S.r.l. at the BCCM/LMG/LMG on 06.03.2025 with the deposit number LMG P-34004; and
vi) at least one bacterial strain belonging to the species *Lactobacillus helveticus,* wherein said strain is the bacterial strain *Lactobacillus helveticus* BD08 deposited by Beingpharma S.r.l. at the BCCM/LMG/LMG on 06.03.2025 with the deposit number LMG P-34003; and
vii) at least one bacterial strain belonging to the species *Bifidobacterium breve,* wherein said strain is the bacterial strain *Bifidobacterium breve* BB02 deposited by Beingpharma S.r.l. at the BCCM/LMG/LMG on 06.03.2025 with the deposit number LMG P-34009; or mixtures thereof; and
viii) vitamin D; preferably vitamin D3.

Preferably the mixture comprises or, alternatively, consists of:
*i) Bifidobacterium animalis* subsp. *lactis* BL03 **(LMG P-34006)** and *Bifidobacterium animalis* subsp. *lactis* BI04 **(LMG P-34007);** and
ii) *Streptococcus thermophilus* BT01 **(LMG P-34002);** and
iii) *Lactiplantibacillus plantarum* BP06 **(LMG P-34005);** and
iv) *Lactobacillus acidophilus* BA05 **(LMG P-34008);** and
*v) Lacticaseibacillus paracasei* BP07 **(LMG P-34004);** and
vi) *Lactobacillus helveticus* BD08 **(LMG P-34003);** and
vii) *Bifidobacterium breve* BB02 **(LMG P-34009);** and
viii) vitamin D; preferably, vitamin D3.

More preferably, the mixture comprises vitamin D3.

In the context of the present invention, the term "mixture" indicates the set of active components which exercise a beneficial effect, preferably a biological effect.

In addition to the above-described components, if desired or necessary, the mixture of the invention can comprise further active components.

Advantageously, the mixture according to the first aspect of the invention (briefly, "mixture of the invention") and/or a composition comprising it are capable of reducing intestinal permeability in subjects with a diagnosis of irritable bowel syndrome without constipation (see the clinical study in the experimental section).

According to the invention, said mixture and/or said composition are capable of reducing intestinal permeability in subjects with a diagnosis of irritable bowel syndrome without constipation, wherein said diagnosis is made according to Rome IV criteria.

In the context of the present invention, the term "intestinal permeability" means, among other things, the ability of the intestinal barrier to prevent the passage, and thus entry into the bloodstream, of bodies such as toxins, bacteria or undigested food residues. Preferably, the intestinal barrier is made up of the intestinal mucosa which, in turn, i) is made up of epithelial cells, immune cells and the secretions thereof and ii) is selectively permeable, that is, it allows the absorption of nutrients and prevents the absorption of said bodies such as toxins, bacteria or undigested food residues. The gut microbiota contributes to regulating the integrity and function of the intestinal barrier. It is well known that increased intestinal permeability is a condition in which the intestinal barrier is altered and allows bodies such as toxins, bacteria or undigested food residues to pass through it, thus causing abdominal pain and visceral hypersensitivity. The condition of increased intestinal permeability is correlated with irritable bowel syndrome (IBS).

In the context of the present invention, the term "disorders associated therewith" in the wording "irritable bowel syndrome and/or disorders associated therewith" means, for example, physical health disorders deriving from or correlated with irritable bowel syndrome (IBS) and preferably selected from the group comprising or, alternatively consisting of diarrhoea, including diarrhoea with frequent bowel movements, diarrhoea with soft stools, diarrhoea with liquid stools and diarrhoea associated with urgency, alternation of periods with diarrhoea and constipation, bowel irregularity, and dysbiosis.

A second aspect of the present invention relates to a pharmaceutical and/or nutraceutical composition comprising the mixture according to the first aspect of the invention and excipients and/or additives of food grade or pharmaceutical grade.

In the context of the present invention, the term "composition" is meant to indicate the set of active components present in the mixture and the additives and/or excipients and/or further components that are not active components. Even if not expressly indicated, the term "composition" in the present description includes pharmaceutical compositions, nutraceutical compositions, food compositions or compositions for medical devices as per Regulation (EU) 2017/745 (briefly, the composition(s) of the present invention) or compositions for supplements and foods for special medical purposes (FSMPs).

Unless otherwise specified, the indication that a composition "comprises" one or more components means that other components may also be present in addition to the one or ones specifically indicated, even where they have not necessarily been expressly indicated, i.e. the composition may also contain exclusively those components, and the indication that a composition "consists" of certain components means that the presence of other components is excluded.

Advantageously, the mixture according to the invention and/or the composition according to the invention comprise or consist of natural ingredients, which thus make both the mixture and the pharmaceutical and/or nutraceutical composition comprising that mixture free of side effects and characterised by high tolerability.

Advantageously, the mixture as defined above and the composition comprising it can be advantageously used as a medicament or as a food, preferably for reducing intestinal dysbiosis, and/or in a method of treatment, preventive or curative, of irritable bowel syndrome and/or disorders associated therewith.

In the context of the present invention, the term "food" means, for example, a food preparation, preferably capable of providing health benefits, more preferably capable of providing health benefits selected from the group comprising or, alternatively, consisting of: maintaining the health (i.e. a physiological condition) of the intestinal barrier and/or gut microbiota.

Furthermore, each of said bacterial strains i) - vii) as indicated above can also be used, individually or together with other bacterial strains i) - vii) as specified above, as a medicament or as a food, preferably for use in reducing intestinal dysbiosis and/or for use in a method of treatment, preventive or curative, of irritable bowel syndrome and/or disorders associated therewith.

Advantageously, said at least one bacterial strain i) - vii) as specified above is capable of reducing intestinal permeability in subjects with a diagnosis of irritable bowel syndrome without constipation.

According to the invention, said at least one bacterial strain i) - vii) is capable of reducing intestinal permeability in subjects with a diagnosis of irritable bowel syndrome without constipation, wherein said diagnosis is made according to Rome IV criteria.

A last aspect of the present description relates to a method of treatment of irritable bowel syndrome and/or disorders associated therewith which comprises administering to a subject in need the mixture and/or composition according to the invention and/or said at least one bacterial strain i) - vii).

### BRIEF DESCRIPTION OF THE FIGURES

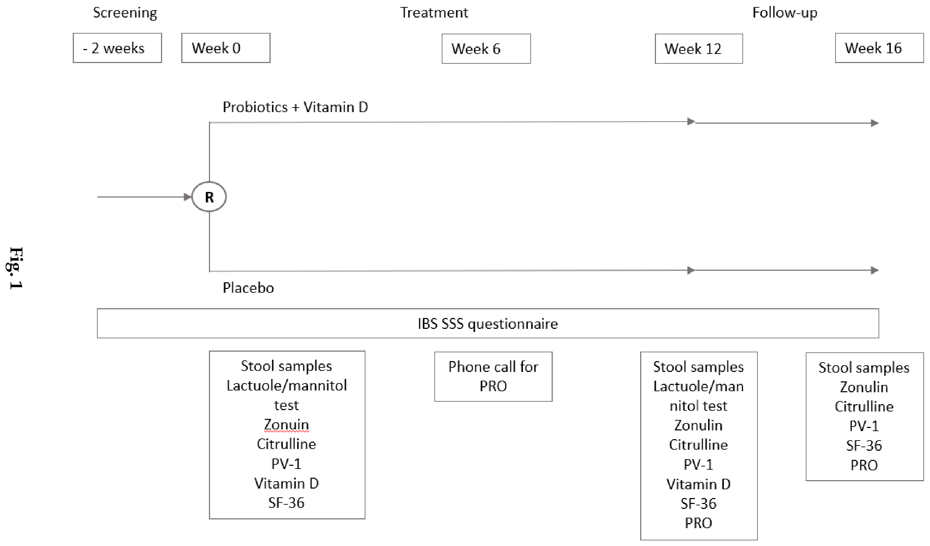

Figure 1: schematic representation of the study design.

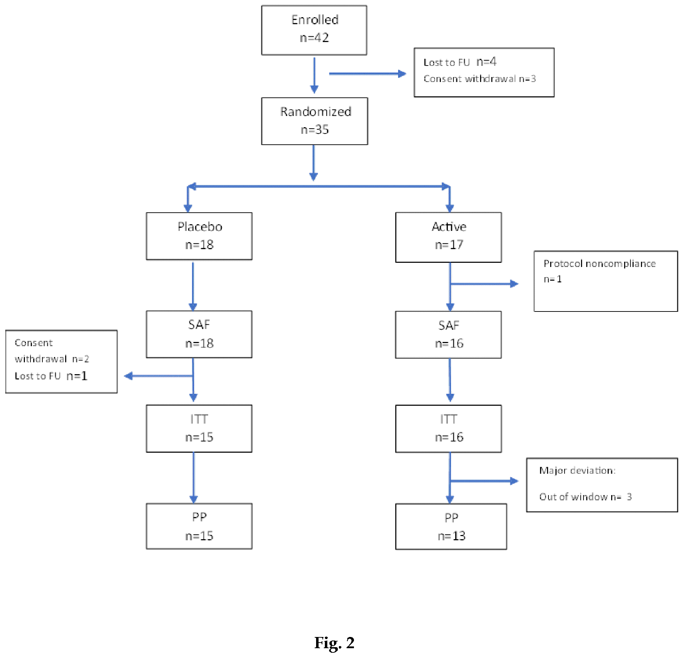

Figure 2: flow diagram of participants according to CONSORT guidelines. 42 patients were enrolled in the study, 3 withdrew their consent and 4 interrupted the study before randomisation, 1 did not take any drug and was excluded from the SAF population, and 3 had no post-baseline data and were excluded from the intention-to-treat (ITT) set. One patient interrupted the study/treatment after the third day and 2 terminated the study before the end. Twenty-eight patients were included in the per-protocol (PP) set.

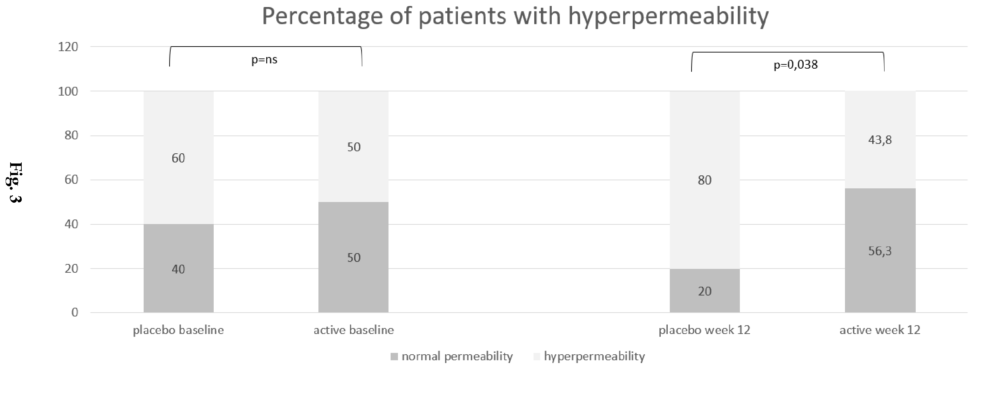

Figure 3: Distribution of patients with normal intestinal permeability (lactulose/mannitol ratio <0.03) and with increased permeability (lactulose/mannitol ratio ≥ 0.03) in the placebo group and in the active group at baseline and at the end of treatment (week 12), expressed as a percentage.

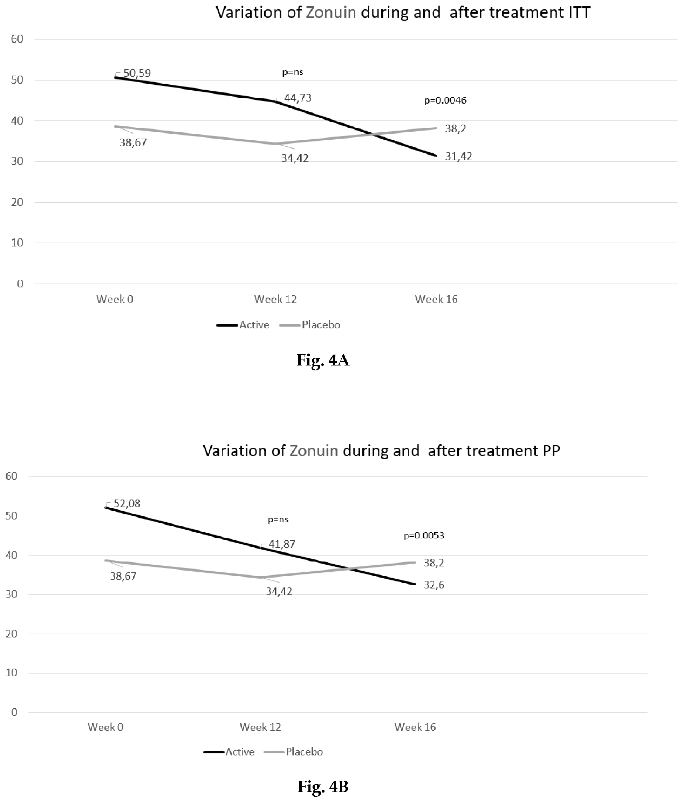

Figure 4: mean zonulin values in the ITT population (A) and PP population (B). The p-values refer to the differences between the active and placebo groups in the mean difference in change in the zonulin levels compared to baseline. See the text for the p-values for the mean difference in within-group change before and after treatment.

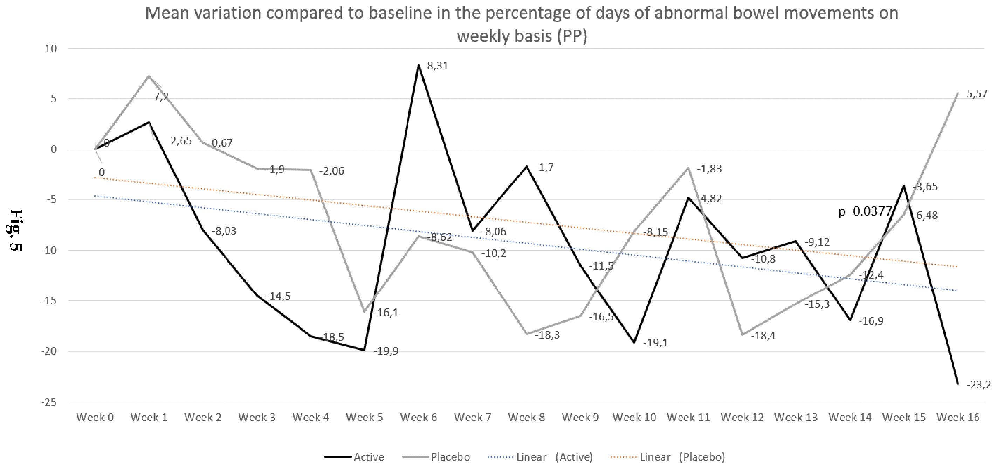

Figure 5: mean percentages of days of abnormal bowel movements on a weekly basis in the PP population. The p-values refer to the mean difference in change between the placebo and active groups (-31.82, p=0.0377) at week 16.

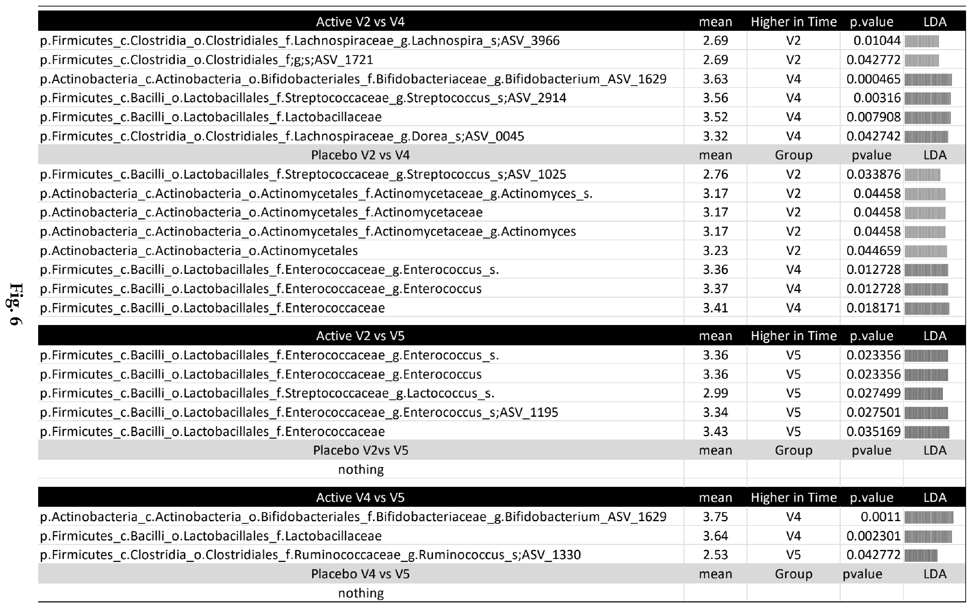

Figure 6. Comparison of microbiota between the treatment timepoints by LEfSE. The figure shows the LEfSE results with the mean values in the group in which it is higher and the LDA for all comparisons between the timepoints (V2, V4, V5) for each treatment (active or placebo). The taxonomy for all variables is provided in the following format: p, phylum; c, class; o, order; f, family; g, genus; s, species; ASV, amplicon sequence variant. The p_Actinobacteria.c_Actinobacteria.o_Bifidobacteriales.f_Bifidobacteriaceae.g_Bifidobacterium.ASV_1629 and p_Firmicutes.c_Bacilli.o_Lactobacillales.f_Streptococcaceae.g_Streptococcus.s_.ASV_2914 sites were manually checked with BLAST and correspondence was found with *Bifidobacterium animalis* subsp. *lactis* and *Streptococcus thermophilus,* respectively.

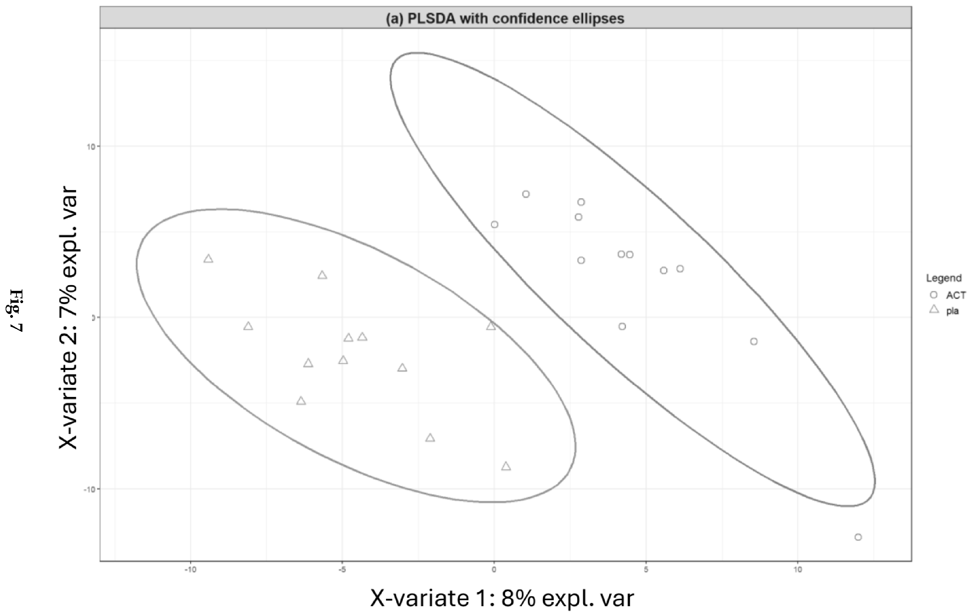

Figure 7: Classification of subjects at point V4 of different treatments by PLSDA. The figure shows the clustering results of partial least squares-discriminant analysis (PLSDA) of the subjects at timepoint V4, comparing two treatments: Active and Placebo. PLSDA was conducted using an equal number of subjects in both treatment groups, for a total of 24 subjects. The subjects who received the active treatment are represented by circles, whereas those who received the placebo treatment are represented by triangles.

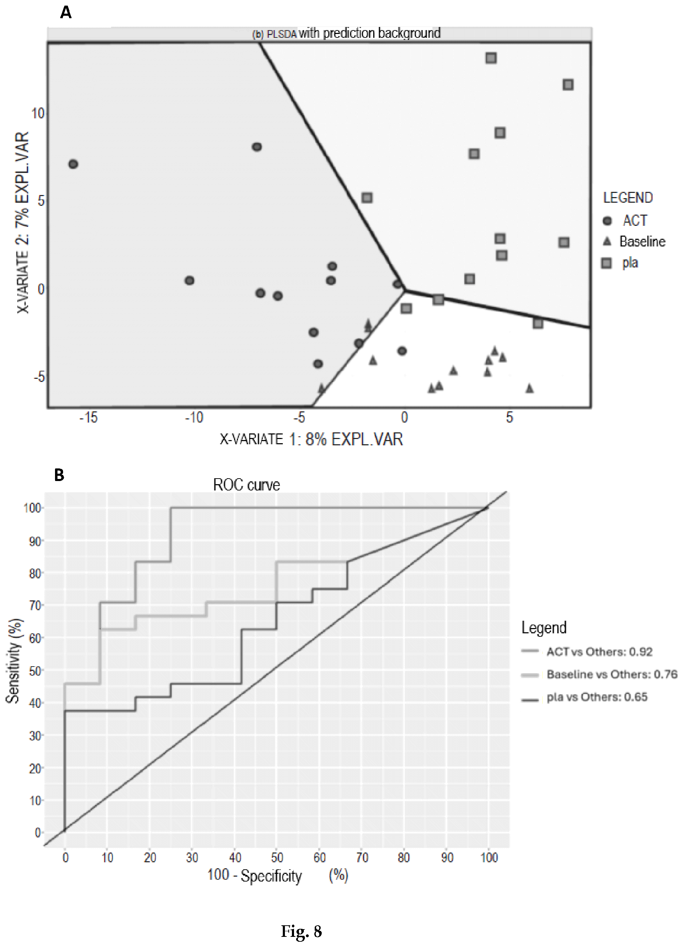

Figure 8 A: Classification of subjects at point V4 of different treatments and at baseline by PLSDA. The figure shows the clustering results of partial least squares-discriminant analysis (PLSDA) of the subjects at timepoint V4 of the two treatments, Placebo and Active, and at point V2, indicated as "Baseline". PLSDA was conducted using an equal number of subjects in both treatment groups, for a total of 36 subjects. The subjects who received the active treatment are represented by dots, whereas those who received the placebo treatment are represented by squares and the subject at baseline is labelled with triangles.
Figure 8 B: the figure shows the ROC curve (the upper curve refers to subjects who received the active treatment vs the other subjects, the lower curve refers to the baseline subject vs the other subjects and the curve interposed between the upper curve and the lower curve refers to the subjects who received the placebo treatment vs the other subjects).

### DETAILED DESCRIPTION

The Applicant, following an intense research activity, selected specific strains of bacteria whose properties are capable of exerting a beneficial effect at the gut level by reducing intestinal dysbiosis.

Advantageously, said strains of bacteria and a mixture comprising them are capable of preventing and/or treating irritable bowel syndrome and/or disorders associated therewith.

The subject matter of the present invention relates to at least one bacterial strain selected in the group which comprises or, alternatively, consists of:
i) at least one bacterial strain belonging to the species *Bifidobacterium animalis* subsp. *lactis,* wherein said strain is selected from the bacterial strain *Bifidobacterium animalis* subsp. *lactis* BL03 deposited by Beingpharma S.r.l. at the BCCM/LMG/LMG on 06.03.2025 with the deposit number **LMG P-34006** and/or the bacterial strain *Bifidobacterium animalis* subsp. *lactis* BI04 deposited by Beingpharma S.r.l. at the BCCM/LMG/LMG on 06.03.2025 with the deposit number **LMG P-34007;** and/or
ii) at least one bacterial strain belonging to the species *Streptococcus thermophilus,* wherein said strain is the bacterial strain *Streptococcus thermophilus* BT01 deposited by Beingpharma S.r.l. at the BCCM/LMG/LMG on 06.03.2025 with the deposit number **LMG P-34002;** and/or
iii) at least one bacterial strain belonging to the species *Lactiplantibacillus plantarum,* wherein said strain is the bacterial strain *Lactiplantibacillus plantarum* BP06 deposited by Beingpharma S.r.l. at the BCCM/LMG/LMG on 06.03.2025 with the deposit number **LMG P-34005;** and/or
iv) at least one bacterial strain belonging to the species *Lactobacillus acidophilus,* wherein said strain is the bacterial strain species *Lactobacillus acidophilus* BA05 deposited by Beingpharma S.r.l. at the BCCM/LMG/LMG on 06.03.2025 with the deposit number **LMG P-34008;** and/or
v) at least one bacterial strain belonging to the species *Lacticaseibacillus paracasei,* wherein said strain is the bacterial strain *Lacticaseibacillus paracasei* BP07 deposited by Beingpharma S.r.l. at the BCCM/LMG/LMG on 06.03.2025 with the deposit number **LMG P-34004;** and/or
vi) at least one bacterial strain belonging to the species *Lactobacillus helveticus,* wherein said strain is the bacterial strain *Lactobacillus helveticus* BD08 deposited by Beingpharma S.r.l. at the BCCM/LMG/LMG on 06.03.2025 with the deposit number **LMG P-34003;** and/or
vii) at least one bacterial strain belonging to the species *Bifidobacterium breve,* wherein said strain is the bacterial strain *Bifidobacterium breve* BB02 deposited by Beingpharma S.r.l. at the BCCM/LMG/LMG on 06.03.2025 with the deposit number **LMG P-34009;** or mixtures thereof;
for use in a method of treatment, preventive or curative, of irritable bowel syndrome and/or disorders associated therewith, more preferably for use in the treatment of irritable bowel syndrome not characterised by constipation in subjects with a diagnosis according to Rome IV criteria.

Furthermore, said at least one bacterial strain i) - vii) is advantageously used in a method for reducing intestinal permeability in subjects with a diagnosis of irritable bowel syndrome without constipation, wherein said diagnosis is made according to Rome IV criteria.

Preferably, the present invention relates to at least one bacterial strain selected from the group that comprises or, alternatively, consists of:
*i) Bifidobacterium animalis* subsp. *lactis BL03* **(LMG P-34006)** and *Bifidobacterium animalis* subsp. *lactis* BI04 **(LMG P-34007)** and a vitamin D; preferably, vitamin D3;
ii) *Streptococcus thermophilus* BT01 **(LMG P-34002)** and a vitamin D; preferably, vitamin D3;
iii) *Lactiplantibacillus plantarum BP06* **(LMG P-34005)** and a vitamin D; preferably, vitamin D3;
iv) *Lactobacillus acidophilus* BA05 **(LMG P-34008)** and a vitamin D; preferably, vitamin D3;
*v) Lacticaseibacillus paracasei BP07* **(LMG P-34004)** and a vitamin D; preferably, vitamin D3;
vi) *Lactobacillus helveticus BD08* **(LMG P-34003)** and a vitamin D; preferably, vitamin D3;
vii) *Bifidobacterium breve BB02* **(LMG P-34009)** and a vitamin D; preferably, vitamin D3, or mixtures thereof for use in a method of treatment, preventive or curative, of irritable bowel syndrome and/or disorders associated therewith; more preferably for use in the treatment of irritable bowel syndrome not characterised by constipation in subjects with a diagnosis according to Rome IV criteria.

All strains of bacteria described in the present invention were deposited by the Applicant, as Depositor, according to the provisions of the Budapest treaty, in the internationally recognised culture collection Belgian Coordinated Collections of Micro-organisms (BCCM) Laboratorium voor Microbiologie - Bacteriënverzameling (LMG), Universiteit Gent at Karel Lodewijk Ledeganckstraat 35, 9000 Ghent, Belgium.

Because of their properties, one or more of said strains can be advantageously used as a medicament or a food. Preferably, said one or more strains are used for reducing intestinal dysbiosis and/or for use in a method of treatment, preventive or curative, of irritable bowel syndrome and/or disorders associated therewith; preferably for use in the treatment of irritable bowel syndrome and/or disorders associated therewith, wherein said diagnosis is made according to Rome IV criteria.

Another aspect of the present invention relates to a mixture comprising or, alternatively, consisting of:
i) at least one bacterial strain belonging to the species *Bifidobacterium animalis* subsp. *lactis,* wherein said strain is selected from the bacterial strain *Bifidobacterium animalis* subsp. *lactis* BL03 deposited by Beingpharma S.r.l. at the BCCM/LMG/LMG on 06.03.2025 with the deposit number **LMG P-34006** and/or the bacterial strain *Bifidobacterium animalis* subsp. *lactis* BI04 deposited by Beingpharma S.r.l. at the BCCM/LMG/LMG on 06.03.2025 with the deposit number **LMG P-34007;** and/or
ii) at least one bacterial strain belonging to the species *Streptococcus thermophilus,* wherein said strain is the bacterial strain *Streptococcus thermophilus* BT01 deposited by Beingpharma S.r.l. at the BCCM/LMG/LMG on 06.03.2025 with the deposit number **LMG P-34002;** and/or
iii) at least one bacterial strain belonging to the species *Lactiplantibacillus plantarum,* wherein said strain is the bacterial strain *Lactiplantibacillus plantarum* BP06 deposited by Beingpharma S.r.l. at the BCCM/LMG/LMG on 06.03.2025 with the deposit number **LMG P-34005;** and/or
iv) at least one bacterial strain belonging to the species *Lactobacillus acidophilus,* wherein said strain is the bacterial strain species *Lactobacillus acidophilus* BA05 deposited by Beingpharma S.r.l. at the BCCM/LMG/LMG on 06.03.2025 with the deposit number **LMG P-34008;** and/or
v) at least one bacterial strain belonging to the species *Lacticaseibacillus paracasei,* wherein said strain is the bacterial strain *Lacticaseibacillus paracasei* BP07 deposited by Beingpharma S.r.l. at the BCCM/LMG/LMG on 06.03.2025 with the deposit number **LMG P-34004;** and/or
vi) at least one bacterial strain belonging to the species *Lactobacillus helveticus,* wherein said strain is the bacterial strain *Lactobacillus helveticus* BD08 deposited by Beingpharma S.r.l. at the BCCM/LMG/LMG on 06.03.2025 with the deposit number **LMG P-34003;** and/or
vii) at least one bacterial strain belonging to the species *Bifidobacterium breve,* wherein said strain is the bacterial strain *Bifidobacterium breve* BB02 deposited by Beingpharma S.r.l. at the BCCM/LMG/LMG on 06.03.2025 with the deposit number **LMG P-34009;** or mixtures thereof; and
viii) a vitamin D, preferably vitamin D3.

Another aspect of the present invention relates to a mixture comprising or, alternatively, consisting of:
i) a bacterial strain belonging to the species *Bifidobacterium animalis* subsp. *lactis,* wherein said strain is the bacterial strain *Bifidobacterium animalis* subsp. *lactis* BL03 deposited by Beingpharma S.r.l. at the BCCM/LMG/LMG on 06.03.2025 with the deposit number **LMG P-34006** and a bacterial strain belonging to the species *Bifidobacterium animalis* subsp. *lactis* wherein said strain is the bacterial strain *Bifidobacterium animalis* subsp. *lactis* BI04 deposited by Beingpharma S.r.l. at the BCCM/LMG/LMG on 06.03.2025 with the deposit number **LMG P-34007;** and
ii) at least one bacterial strain belonging to the species *Streptococcus thermophilus,* wherein said strain is the bacterial strain *Streptococcus thermophilus* BT01 deposited by Beingpharma S.r.l. at the BCCM/LMG/LMG on 06.03.2025 with the deposit number **LMG P-34002;** and
iii) at least one bacterial strain belonging to the species *Lactiplantibacillus plantarum,* wherein said strain is the bacterial strain *Lactiplantibacillus plantarum* BP06 deposited by Beingpharma S.r.l. at the BCCM/LMG/LMG on 06.03.2025 with the deposit number **LMG P-34005;** and
iv) at least one bacterial strain belonging to the species *Lactobacillus acidophilus,* wherein said strain is the bacterial strain species *Lactobacillus acidophilus* BA05 deposited by Beingpharma S.r.l. at the BCCM/LMG/LMG on 06.03.2025 with the deposit number **LMG P-34008;** and
v) at least one bacterial strain belonging to the species *Lacticaseibacillus paracasei,* wherein said strain is the bacterial strain *Lacticaseibacillus paracasei* BP07 deposited by Beingpharma S.r.l. at the BCCM/LMG/LMG on 06.03.2025 with the deposit number **LMG P-34004;** and
vi) at least one bacterial strain belonging to the species *Lactobacillus helveticus,* wherein said strain is the bacterial strain *Lactobacillus helveticus* BD08 deposited by Beingpharma S.r.l. at the BCCM/LMG/LMG on 06.03.2025 with the deposit number **LMG P-34003;** and
vii) at least one bacterial strain belonging to the species *Bifidobacterium breve,* wherein said strain is the bacterial strain *Bifidobacterium breve* BB02 deposited by Beingpharma S.r.l. at the BCCM/LMG/LMG on 06.03.2025 with the deposit number **LMG P-34009;** or mixtures thereof; and
viii) a vitamin D, preferably vitamin D3.

In the context of the present invention and in the appended claims, said strains of bacteria may also be indicated only by their name and their deposit number (e.g. *Bifidobacterium animalis* subsp. *lactis* BL03 **(LMG P-34006)).**

In the context of the present invention, "at least one bacterial strain belonging to the species *Bifidobacterium animalis* subsp. *lactis"* is meant to refer to, and include, any strain of *Bifidobacterium animalis* subsp. *lactis* (including the bacterial strains *Bifidobacterium animalis* subsp. *lactis* BL03 **(LMG P-34006)** and/or *Bifidobacterium animalis* subsp. *lactis* BI04 **(LMG P-34007))** and/or mixtures of strains of *Bifidobacterium animalis* subsp. *lactis,* both in viable form and in viable but non-replicating form, or in non-viable, inactivated, semi-inactivated, or dead form.

In the context of the present invention, the terms "non-viable", "inactivated" or "dead" refer to a microorganism that is no longer able, temporarily or definitively, to form colonies in a culture. "Non-viable", "inactivated" or "dead" microorganisms can have intact or broken cell membranes. Therefore, the term "non-viable", "inactivated" or "dead" also indicates extracts and lysates of microorganisms. In the case of "inactivated" microorganisms, the microorganisms may retain surface structures (lipids, proteins) and molecules within the biomass that are still active and capable of interacting and bringing probiotic functions without having, however, an active metabolism. Inactivated microorganisms can be produced with any method known to the person skilled in the art, such as irradiation (with gamma rays, X-rays, exposure to UV rays) or exposure to heat. The type of treatment, intensity, dose and exposure time are adjusted by the person skilled in the art based on the quantity and nature of the probiotic microorganisms to be inactivated.

In the context of the present invention, *Bifidobacterium animalis* subsp. *lactis* BL03 **(LMG P-34006)** and/or *Bifidobacterium animalis* subsp. *lactis* BI04 **(LMG P-34007)** are preferably in viable or viable but non-replicating form.

In the context of the present invention, "at least one bacterial strain belonging to the species *Streptococcus thermophilus",* is meant to refer to, and include, any strain of *Streptococcus thermophilus* (including the bacterial strain *Streptococcus thermophilus* BT01 **(LMG P-34002))** and/or mixtures of strains of *Streptococcus thermophilus* both in viable form and in viable but non-replicating form, or in non-viable, inactivated, semi-inactivated, or dead form.

In the context of the present invention, said *Streptococcus thermophilus* BT01 **(LMG P-34002)** is preferably in viable or viable but non-replicating form.

In the context of the present invention, "at least one bacterial strain belonging to the species *Lactiplantibacillus plantarum"* is meant to refer to, and include, any strain of *Lactiplantibacillus plantarum* (including the bacterial strain *Lactiplantibacillus plantarum* BP06 **(LMG P-34005))** both in viable form and in viable but non-replicating form, or in non-viable, inactivated, semi-inactivated, or dead form.

In the context of the present invention, said *Lactiplantibacillus plantarum* BP06 **(LMG P-34005)** is preferably in viable or viable but non-replicating form.

In the context of the present invention, "at least one bacterial strain belonging to the species *Lactobacillus acidophilus"* is meant to refer to, and include, any strain of *Lactobacillus acidophilus* (including the bacterial strain *Lactobacillus acidophilus* BA05 **(LMG P-34008))** both in viable form and in viable but non-replicating form, or in non-viable, inactivated, semi-inactivated, or dead form.

In the context of the present invention, said *Lactobacillus acidophilus* BA05 **(LMG P-34008)** is preferably in viable or viable but non-replicating form.

In the context of the present invention, "at least one bacterial strain belonging to the species *Lacticaseibacillus paracasei"* is meant to refer to, and include, any strain of *Lacticaseibacillus paracasei* (including the bacterial strain *Lacticaseibacillus paracasei* BP07 **(LMG P-34004))** both in viable form and in viable but non-replicating form, or in non-viable, inactivated, semi-inactivated, or dead form.

In the context of the present invention, said *Lacticaseibacillus paracasei* BP07 **(LMG P-34004)** is preferably in viable or viable but non-replicating form.

In the context of the present invention, "at least one bacterial strain belonging to the species *Lactobacillus helveticus"* is meant to refer to, and include, any strain of *Lactobacillus helveticus* (including the bacterial strain *Lactobacillus helveticus* BD08 **LMG P-34003))** both in viable form and in viable but non-replicating form, or in non-viable, inactivated, semi-inactivated, or dead form.

In the context of the present invention, said *Lactobacillus helveticus* BD08 **(LMG P-34003)** is preferably in viable or viable but non-replicating form.

In the context of the present invention, "at least one bacterial strain belonging to the species *Bifidobacterium breve"* is meant to refer to, and include, any strain of *Bifidobacterium breve* (including the bacterial strain *Bifidobacterium breve* BB02 **(LMG P-34009))** both in viable form and in viable but non-replicating form, or in non-viable, inactivated, semi-inactivated, or dead form.

In the context of the present invention, said *Bifidobacterium breve* BB02 **(LMG P-34009)** is preferably in viable or viable but non-replicating form.

Furthermore, for example, one or more of the strains described in the present invention can be in pasteurised form. Pasteurisation is a heat sanitisation process applied to some foods with the aim of minimising the health risks due to heat-sensitive pathogenic microorganisms, such as bacteria in vegetative form, fungi and yeasts, with a minimal alteration of the chemical, physical and organoleptic characteristics of the food.

Said one or more strains can be subjected to pasteurisation by means of known methods.

For example, the pasteurisation can be carried out with a heat treatment preferably at temperatures comprised from 60°C to 90°C for a time comprised from 1 second to 3600 seconds, preferably from 10 seconds to 2700 seconds, even more preferably from 60 seconds to 1200 seconds.

Preferably, the mixture according to the invention comprises both strains of bacteria belonging to the species *Bifidobacterium animalis* subsp. *lactis.* In other words, the mixture according to the invention comprises both *Bifidobacterium animalis* subsp. *lactis* BL03 **(LMG P-34006)** and the bacterial strain *Bifidobacterium animalis* subsp. *lactis* BI04 **(LMG P-34007).**

More preferably, the mixture according to the invention comprises at least one of the bacterial strains selected from:
*Bifidobacterium animalis* subsp. *lactis* BL03 **(LMG P-34006);** and/or
*Bifidobacterium animalis* subsp. *lactis* BI04 **(LMG P-34007);** and/or
*Streptococcus thermophilus* BT01 **(LMG P-34002);** and/or
*Lactiplantibacillus plantarum* BP06 **(LMG P-34005),** and
viii) vitamin D, preferably vitamin D3.

Even more preferably, the mixture according to the invention comprises or, alternatively, consists of the following bacterial strains:
*i) Bifidobacterium animalis* subsp. *lactis* BL03 **(LMG P-34006)** and *Bifidobacterium animalis* subsp. *lactis* BI04 **(LMG P-34007);** and
ii) *Streptococcus thermophilus* BT01 **(LMG P-34002);** and
iii) *Lactiplantibacillus plantarum* BP06 **(LMG P-34005);** and
iv) *Lactobacillus acidophilus* BA05 **(LMG P-34008);** and
*v) Lacticaseibacillus paracasei* BP07 **(LMG P-34004);** and
vi) *Lactobacillus helveticus* BD08 **(LMG P-34003);** and
vii) *Bifidobacterium breve* BB02 **(LMG P-34009);** and
viii) vitamin D, preferably vitamin D3.

Preferably, one or more of said bacterial strains i) - vii) as specified above can each be in a concentration comprised from 1x10⁵ CFU/g to 1x10¹³ CFU/g, more preferably from 1x10⁶ CFU/g to 1x10¹² CFU/g.

More preferably, the mixture according to the invention preferably comprises or, alternatively, consists of:
*i) Bifidobacterium animalis* subsp. *lactis* BL03 **(LMG P-34006)** and *Bifidobacterium animalis* subsp. *lactis* BI04 **(LMG P-34007);** and
ii) *Streptococcus thermophilus* BT01 **(LMG P-34002);** and
iii) *Lactiplantibacillus plantarum* BP06 **(LMG P-34005);** and
iv) *Lactobacillus acidophilus* BA05 **(LMG P-34008);** and
*v) Lacticaseibacillus paracasei* BP07 **(LMG P-34004);** and
vi) *Lactobacillus helveticus* BD08 **(LMG P-34003);** and
vii) *Bifidobacterium breve* BB02 **(LMG P-34009);** and
viii) vitamin D3.

In fact, as shown in the experimental section, the mixture according to the invention combined with vitamin D3, ("active treatment" or in the figures indicated as "active") significantly modulated the composition of the microbiome, with an increase in *Bifidobacterium animalis* subsp. *lactis* and *Streptococcus thermophilus* (p < 0.05), whereas *Lachnospira* decreased (p < 0.05). The analysis of short-chain fatty acids (SCFAs) showed an increase in lactate (p < 0.01) and acetate (p < 0.05) after the treatment.

In the "active" group, significant changes in *Actinobacteria* and *Proteobacteria* were observed (p < 0.05). Machine learning approaches classified the subjects with an accuracy of 85% and 80%, based on the treatment. Correlation analysis indicated significant associations between microbial biomarkers and clinical parameters (p < 0.05).

The Applicant has advantageously observed that the mixture integrating the bacterial strains i) - vii) as specified above together with vitamin D3 is capable of improving intestinal barrier function through the modulation of the gut microbiota and reduction of intestinal permeability, even after suspension of the treatment, in subjects with IBS without constipation. The mixture according to the invention and, optionally, excipients and/or additives of acceptable food grade or pharmaceutical grade, can be advantageously used as a medicament or a food. Preferably, the mixture can be used to reduce intestinal dysbiosis and/or in a method of treatment, preventive or curative, of irritable bowel syndrome and/or disorders associated therewith; more preferably it is used in the treatment of irritable bowel syndrome and/or disorders associated therewith.

A further aspect of the present invention relates to a composition comprising the mixture according to the invention and, optionally, excipients and/or additives of acceptable food grade or pharmaceutical grade.

Preferably, said mixture is present in said composition in an amount by weight comprised from 10% to 90%, preferably from 25% to 75%, more preferably from 40% to 60%, whereas said at least one conventional carrier and/or excipient are present in an amount by weight comprised from 90% to 10%, preferably from 75% to 25%, more preferably from 60% to 40% by weight, relative to the total weight of the composition.

Preferably, the composition according to the invention comprises a mixture comprising:
*i) Bifidobacterium animalis* subsp. *lactis* BL03 **(LMG P-34006)** and *Bifidobacterium animalis* subsp. *lactis* BI04 **(LMG P-34007);** and
ii) *Streptococcus thermophilus* BT01 **(LMG P-34002);** and
iii) *Lactiplantibacillus plantarum* BP06 **(LMG P-34005);** and
iv) *Lactobacillus acidophilus* BA05 **(LMG P-34008);** and
*v) Lacticaseibacillus paracasei* BP07 **(LMG P-34004);** and
vi) *Lactobacillus helveticus* BD08 **(LMG P-34003);** and
vii) *Bifidobacterium breve* BB02 **(LMG P-34009);** and
viii) vitamin D3;
and, optionally, excipients and/or additives of acceptable food grade or pharmaceutical grade.

Example of compositions are shown in Tables 1-3.

Preferably, said composition comprises:
- *Bifidobacterium animalis* subsp. *lactis* BL03 **(LMG P-34006)** in a percentage amount by weight relative to the total of the bacterial mixture present in said composition comprised from 10% to 50%, preferably from 20% to 40%, more preferably from 30% to 35%; for example 35%;
- *Bifidobacterium. animalis* subsp. *lactis* BI04 **(LMG P-34007)** in a percentage amount by weight relative to the total of the bacterial mixture present in said composition comprised from 5% to 30%, preferably from 10% to 25%, more preferably from 15% to 20%; for example 17.5%;
- *Streptococcus thermophilus* BT01 **(LMG P-34002)** in a percentage amount by weight relative to the total of the bacterial mixture present in said composition comprised from 15% to 40%, preferably from 20% to 35%, more preferably from 25% to 30%; for example 28%;
- *Lactiplantibacillus plantarum* BP06 **(LMG P-34005)** in a percentage amount by weight relative to the total of the bacterial mixture present in said composition comprised from 5% to 30%, preferably from 10% to 25%, more preferably from 13% to 20%; for example 15%;
- *Lactobacillus acidophilus* BA05 **(LMG P-34008)** in a percentage amount by weight relative to the total of the bacterial mixture present in said composition comprised from 1% to 5%, preferably from 2% to 4%, more preferably from 2.5% to 3%; for example 2.8%;
- *Lacticaseibacillus paracasei* BP07 **(LMG P-34004)** in a percentage amount by weight relative to the total of the bacterial mixture present in said composition comprised from 0.5% to 5%, preferably from 0.8% to 3%, more preferably from 1% to 2%; for example 1%;
- *Lactobacillus helveticus* BD08 **(LMG P-34003)** in a percentage amount by weight relative to the total of the bacterial mixture present in said composition comprised from 0.001% to 0.5%, preferably from 0.001% to 0.2%, more preferably from 0.05% to 0.1%; for example 0.07%;
- *Bifidobacterium breve* BB02 **(LMG P-34009)** in a percentage amount by weight relative to the total of the bacterial mixture present in said composition comprised from 0.0001% to 0.01%, preferably from 0.001% to 0.009%, more preferably from 0.002% to 0.005%; for example 0.004%; and
- vitamin D3 from 200 IU to 2500 IU, preferably from 400 IU to 2200 IU, more preferably from 500 IU to 2000 IU and, optionally, excipients and/or additives of acceptable food grade or pharmaceutical grade.

The composition according to the invention is, for example, a pharmaceutical composition or a nutraceutical composition or a composition for supplements or a food composition or a composition for medical devices, for example according to Regulation (EU) 2017/745, or a composition for foods for special medical purposes, for example according to Regulation (EU) No 609/2013 (briefly, the compositions of the invention).

In the context of the present invention, the expression "at least one excipient and/or additive of pharmaceutical grade or food grade" means a substance devoid of therapeutic activity, suitable for pharmaceutical use or food use. In the context of the present invention, the acceptable additives and/or excipients for pharmaceutical or food use comprise all the auxiliary substances known to the person skilled in the art for preparing compositions in solid, semisolid or liquid form, e.g. emulsifiers, diluents, solvents, solubilizers, acidifiers, thickening agents, sweeteners, flavour enhancers, colourants, lubricants, surfactants, preservatives, pH stabilizing buffers and mixtures thereof. According to the invention, said composition is formulated for oral administration, preferably in the form of a capsule, a tablet, a sachet, a stick, an orodispersible stick, a gel for oral use or granules, or as a food.

The composition comprising the mixture according to the invention and, optionally, excipients and/or additives of acceptable food grade or pharmaceutical grade can be advantageously used as a medicament or a food.

Preferably, the composition can be used for reducing intestinal dysbiosis and/or in a method of treatment, preventive or curative, of irritable bowel syndrome and/or disorders associated therewith; more preferably it is used in the treatment of irritable bowel syndrome and/or disorders associated therewith.

Disorders associated with irritable bowel syndrome include, for example, lower abdominal pain, abdominal cramps, meteorism, gas and constipation or diarrhoea, and mucus in stools.

Furthermore, it was observed that the mixture according to the first aspect of the invention and/or a composition comprising it are capable of reducing intestinal permeability in subjects with a diagnosis of irritable bowel syndrome without constipation (see the clinical study in the experimental section).

According to the invention, said mixture and/or said composition are capable of reducing intestinal permeability in subjects with a diagnosis of irritable bowel syndrome without constipation, wherein said diagnosis is made according to Rome IV criteria.

### EXPERIMENTAL SECTION

### Example 1

A composition was prepared in the form of a sachet containing:

**Table 1**

| **Ingredients** | **% w/w** | **Billions/dose** |
|---|---|---|
| *Bifidobacterium animalis* subsp. *lactis* BL03 **(LMG P-34006)** | 35.09% | 175 |
| *Bifidobacterium animalis* subsp. *lactis* BI04 **(LMG P-34007)** | 17.54% | 88 |
| *Streptococcus thermophilus* BT01 **(LMG P-34002)** | 28.07% | 140 |
| *Lactiplantibacillus plantarum* BP06 **(LMG P-34005)** | 15.37% | 77 |
| *Lactobacillus acidophilus* BA05 **(LMG P-34008)** | 2.81% | 14 |
| *Lacticaseibacillus paracasei* BP07 **(LMG P-34004)** | 1.05% | 5.3 |
| *Lactobacillus helveticus* BD08 **(LMG P-34003)** | 0.07% | 0.33 |
| *Bifidobacterium breve* BB02 **(LMG P-34009)** | 0.004% | 0.017 |
| Total bacterial mix | 100% | 500 |
| Vitamin D3 (2000 IU) | | |

and, optionally, excipients and/or additives of acceptable food grade or pharmaceutical grade.

### Example 2

A composition was prepared in the form of a capsule containing:

**Table 2**

| **Ingredients** | **% w/w** | **Billions/dose** |
|---|---|---|
| *Bifidobacterium animalis* subsp. *lactis* BL03 **(LMG P-34006)** | 35.09% | 31 |
| *Bifidobacterium animalis* subsp. *lactis* BI04 **(LMG P-34007)** | 17.54% | 16 |
| *Streptococcus thermophilus* BT01 **(LMG P-34002)** | 28.07% | 25 |
| *Lactiplantibacillus plantarum* BP06 **(LMG P-34005)** | 15.37% | 14 |
| *Lactobacillus acidophilus* BA05 **(LMG P-34008)** | 2.81% | 2.5 |
| *Lacticaseibacillus paracasei* BP07 **(LMG P-34004)** | 1.05% | 0.95 |
| *Lactobacillus helveticus* BD08 **(LMG P-34003)** | 0.07% | 0.06 |
| *Bifidobacterium breve* BB02 **(LMG P-34009)** | 0.004% | 0.003 |
| Total bacterial mix | 100% | 90 |
| Vitamin D3 (500 IU) | | |

and, optionally, excipients and/or additives of acceptable food grade or pharmaceutical grade.

### Example 3

A composition was prepared in billions per gram containing:

**Table 3**

| **Ingredients** | **% w/w** | **Billions/gram** |
|---|---|---|
| *Bifidobacterium animalis* subsp. *lactis* BL03 **(LMG P-34006)** | 35.09% | 100 |
| *Bifidobacterium animalis* subsp. *lactis* BI04 **(LMG P-34007)** | 17.54% | 50 |
| *Streptococcus thermophilus* BT01 **(LMG P-34002)** | 28.07% | 80 |
| *Lactiplantibacillus plantarum* BP06 **(LMG P-34005)** | 15.37% | 43.8 |
| *Lactobacillus acidophilus* BA05 **(LMG P-34008)** | 2.81% | 8 |
| *Lacticaseibacillus paracasei* BP07 **(LMG P-34004)** | 1.05% | 3 |
| *Lactobacillus helveticus* BD08 **(LMG P-34003)** | 0.07% | 0.19 |
| *Bifidobacterium breve* BB02 **(LMG P-34009)** | 0.004% | 0.01 |
| Total bacterial mix | 100% | 285 |
| Vitamin D3 (1000 IU) | | |

and, optionally, excipients and/or additives of acceptable food grade or pharmaceutical grade.

### CLINICAL STUDY

### 1. Materials and methods

### Study design

The Applicant carried out a competitive, multicentre, parallel-group, placebo-controlled, randomised double-blind phase IIb study.

The study provided for a 4-week screening phase (V1), a baseline visit (V2), a 12-week treatment phase (V3) and a further 4-week follow-up (visit at week 16, V4) (Figure 1).

After the signing of the informed consent form, the patients were randomised 1:1 and received one sachet per day of the active product (mixture according to the invention indicated in Table 1) or a placebo for 12 weeks. During the screening phase V1, data on the medical history were collected, the inclusion and exclusion criteria were checked and the IBS-SSS questionnaire was given to the patients. The patients were instructed to fill out an IBS-SSS questionnaire every week, and a Bristol Stool Form Scale questionnaire every day for the entire duration of the study. During the baseline visit V2, patients were asked to provide a stool sample for an analysis of the gut microbiome and short-chain fatty acids (SCFAs; acetate, butyrate, propionate), and a blood sample for an assessment of serum biomarkers and plasma vitamin D levels. Furthermore, the patients were asked to do a lactulose/mannitol (LaMa) test and fill out the SF-36 questionnaire.

They were then randomised to receive the treatment or the placebo.

Thirty-five patients were successfully randomised (28 were included in the per-protocol population; PP).

The randomisation list, with a block size = 4, was computer generated. At the time of randomisation, the centres assigned the kit of the investigational product (IP) to the patient.

Said investigational product IP comprises the probiotic mixture containing the eight viable and lyophilised bacterial strains according to the invention and vitamin D3.

For the sake of brevity, in the clinical study described below the probiotic mixture containing the eight viable and lyophilised bacterial strains according to the invention and vitamin D is indicated by the initials "IP".

After 6 weeks of treatment, the experimenters checked the outcomes reported by the patients, any adverse events (AEs) and concomitant drugs by means of a phone call. At week 12, the patients returned to the centres for a new clinical assessment and for the collection of stool, blood and urine samples for an analysis of the gut microbiome and SCFAs, serum biomarkers and plasma levels of vitamin D and the lactulose/mannitol (LaMa) test, respectively. The quality of life and outcomes reported by patients were also reassessed at the end of treatment. Four weeks after interruption of treatment (16th week), the patients were clinically assessed in relation to the IBS symptoms and quality of life correlated with IBS (follow-up). During the follow-up visit, the patients also had a serum sample collected for an analysis of biomarkers and the gut microbiome and SCFAs in the stools. The side effects were constantly monitored for the whole duration of the study.

### Study population

This study was conducted on a population of adults affected by irritable bowel syndrome without constipation, defined according to Rome IV criteria (Mearin F, Lacy BE, Chang L et al. Bowel Disorders. Gastroenterology 2016, DOI: 10.1053/j.gastro.2016.02.031) aged between 18 and 65 years, monitored in two centres of reference in Italy: Fondazione Policlinico Universitario A. Gemelli IRCCS in Rome and IRCCS Azienda Ospedaliero Universitaria di Bologna in Bologna. The patients were placed on a diet two months before the screening visit and throughout the whole study. The exclusion criteria were constipation-predominant IBS (IBS-C) or unclassified IBS (IBS-U), according to Rome IV criteria. The exclusion criteria included patients with any relevant organic, systemic or metabolic disease (in particular a significant history of heart, kidney, neurological, oncological, endocrinological, metabolic or liver disease), or with ascertained unstable psychiatric conditions or ascertained organic intestinal diseases; previous major abdominal surgical interventions (with the exception of a previous appendectomy); use of antisecretory drugs, including all proton pump inhibitors and H2-antagonists in the two months prior to screening; introduction or modification of the dose of other drugs that influence intestinal permeability in the last 4 weeks prior to screening; taking of probiotics or topical and/or systemic antibiotic therapy in the last 2 months; pregnancy. This study was conducted according to the principles of the Declaration of Helsinki and Good Clinical Practice, after IEC approval of both centres (Protocol 41259/19 ID:2817, meeting of 24/10 /2019), (25/2020/Sper/AOUBo, meeting of 23/01/2020).

### Study products

The IP is a combination of a probiotic consortium (OttaBac^{®}) at a high concentration (500 billion CFU/sachet) and vitamin D (2000 IU of cholecalciferol/sachet). OttaBac^{®} is a probiotic mixture containing the eight viable and lyophilised bacterial strains in the mixture according to the invention: *Bifidobacterium animalis* subsp. *lactis* BL03, *Streptococcus thermophilus* BT01, *Bifidobacterium. animalis* subsp. *lactis* BI04, *Lactiplantibacillus plantarum* BP06, *Lactobacillus acidophilus* BA05, *Lacticaseibacillus paracasei* BP07, *Lactobacillus helveticus* BD08 and *Bifidobacterium breve* BB02. The patients were instructed to take the product with water on an empty stomach. The placebo contained maltose and maize starch. The active product and placebo were packaged in sachets that were identical and indistinguishable based on the organoleptic properties.

### Study results

The aims of the study were to:
1) assess the effect of the IP on intestinal permeability *in vivo,* analysed by measuring the urinary excretion of orally administered lactulose and mannitol;
2) assess the effect of the IP on intestinal barrier function, analysed through serum biomarkers, including zonulin,
3) assess the effect of the IP on the composition and function of the gut microbiota, analysed through 16S-rRNA characterisation, the prediction of functional pathways based on PICRUSt and metabolic activity based on the faecal concentration of short-chain fatty acids (SCFAs; acetate, butyrate, propionate);
4) assess the effect of the IP on irritable bowel syndrome (IBS) symptoms based on the data of the IBS-SSS questionnaire and Bristol Stool Form Scale;
5) assess the effect of the IP on quality of life via the SF-36;
6) assess the effect of the IP on serum vitamin D levels.

### Statistical analysis

As this was a physio-pathological study, no calculations were performed to identify the number of patients necessary to detect statistical significance. In fact, there do not exist any previously conducted reference studies to which reference may be made for statistical power. On the basis of feasibility criteria and previously published studies with similar physio-pathological endpoints, 48 patients were included in the study.

The study population for the analysis of the effectiveness variables (intention-to-treat analysis, ITT) included all the patients who received at least one dose of the study treatment and had at least one assessment of the post-baseline diary. In the analysis of the results for the ITT population, when there were more than 2 timepoints, the missing data were not replaced but rather interpolated with mixed models. When there were only two timepoints, the last observation carried forward (LOCF) method was used to impute the data.

The weekly frequency and consistency of stools, assessed using the Bristol Stool Form Scale, were calculated as the mean of daily data. The baseline for the diary data was the daily mean of 2 weeks (based on available data) before randomisation. Bowel movements were considered normal if the Bristol Stool Form Scale was < 5 and the frequency of bowel movements was ≤ 3/die, otherwise they were considered "abnormal".

The percentage of days in a week with abnormal bowel movements was also calculated.

Frequencies, means, standard deviations and intervals were used as descriptive statistics. In the case of categorical variables, the absolute counts (n) and percentages (%) were presented for every category. Depending on the cases, the chi-squared test with Yate's correction, Mann-Whitney and Wilcoxon tests were applied. The odds ratio, together with the 95% confidence interval, was used to compare the response to the two treatments. Furthermore, covariance analysis, including the study group factors and the baseline value, was used to assess the between-group differences.

A two-tailed p-value of less than 0.05 was considered statistically significant. The data were analysed using the SPSS/PC+ statistical package.

### Lactulose/mannitol test

The urinary LaMa test is a test that has been amply described in the literature for the assessment of intestinal permeability, because of the different characteristics of the two sugars involved: mannitol is a probe of small dimensions that can permeate the intestinal barrier through both the paracellular and transcellular pathways; lactulose, by contrast, is too large to permeate the small transcellular pathways and selectively permeate through the paracellular one. Considering that mannitol has few paracellular pathways compared to transcellular ones, it effectively measures the transcellular pathways and, finally, its permeation rate is directly correlated to the intestinal surface. In contrast, lactulose only permeates the damaged area of the same intestinal surface. Finally, the ratio of excretion of lactulose-mannitol in urine provides an indirect estimation of intestinal permeability and its increase is correlated with an increase in intestinal permeability.

The test was sent off and performed centrally for all centres at the same laboratory appointed to perform mass spectrometry for quantification of the urinary concentrations of the two sugars. At the time of screening, the patients were provided with a urinary container and were instructed on how to correctly collect a urine sample. On the day of the baseline visit and at the 12th week, before going to the centre, the patients collected the first urine of the morning, adding chlorhexidine to the sample in a first container. At the centre, a LaMa syrup was administered to the patients, who were instructed to collect all urine over the next 6 hours in a second container, adding chlorhexidine. Two test tubes were drawn from the two containers, respectively, and stored at +2/+8 °C before being sent off and analysed within 2 weeks after collection. LaMa ratios ≧ 0.03 were considered consistent with hyperpermeability.

### Zonulin and vitamin D levels

The serum biomarker zonulin was evaluated.

Zonulin is the endogenous counterpart of the *Vibrio cholerae* enterotoxin and to date is the only human protein known to be capable of reversibly opening the tight junctions. An increase in the expression of zonulin has been correlated with an increase in intestinal permeability and may be considered a reliable marker in various gastrointestinal disorders, including inflammatory bowel diseases, IBS and coeliac disease. The serum biomarkers were evaluated at baseline, at the 12th week and at the 16th week, whereas vitamin D was evaluated only at baseline and at the 12th week. Blood samples were collected by venepuncture and centrifuged at 2000 g for 10 minutes at 18-25° C to separate the serum. The serum was stored at -80 °C for zonulin and at -20 °C to evaluate vitamin D. The samples were analysed within two months after their collection. For the analysis of zonulin, the ELISA test was used. The levels of 25-hydroxycholecalciferol were measured in the serum. Zonulin values comprised between 20 and 48 ng/ml were considered normal.

### Ecological and functional characterisation of gut microbiota and analysis of SCFAs

Stool samples were collected at baseline (V2), at week 12 (V4) and at week 16 (V5), with a tolerance of ± 4 days, for metataxonomic analysis of the 16S rRNA gene V3-V4. These samples were stored at -80 °C until DNA extraction in the centres for the duration of the study. Once all the stool samples had been collected, they were sent under refrigerated conditions to the centralised laboratory (De FENS) appointed to carry out GM profiling and the SCFA analyses.

For the extraction process, the stools were thawed at +4 °C and vigorously mixed with a sterile spatula for 2-3 minutes. Subsequently, 150 mg of stools were weighed and treated with the PowerSoil Pro kit (Qiagen) according to the manufacturer's instructions. The extracted DNA was quantified with the Qubit Broad Range kit (Thermo Fisher Scientific, Waltham, MA, USA) and used for metataxonomic analysis through 16S rRNA gene profiling. The NovaSeq 6000 platform with 2 × 250 bp sequencing (NovaSeq 6000 SP Reagent Kit, 5 Gbasi) was used to sequence the amplicons of the 16S rRNA gene, aiming at the V3 and V4 variable regions. These amplicons were obtained using the primers 341F (SEQ:ID:No.1) (5'-CCTACGGGNBGCASCAG-3') and 805R (SEQ:ID:No.2) (5'-GACTACNVGGGTATCTAATCC-3') (BMR Genomics, Padova, Italy). The sequencing readings were processed using the Quantitative Insights Into Microbial Ecology 2 (QIIME2) bioinformatics pipeline, version 2023.2 and employing the Divisive Amplicon Denoising Algorithm (DADA2). For taxonomic assignment to the amplicon sequence variants (ASVs), the Greengenes database version 13_8 was used. In order to minimise technological biases, the 16S rRNA gene profile analysis was conducted simultaneously for all the stool samples of this study. A BMR Genomics mock community was inserted between the samples to validate the output after processing of the readings. The raw metataxonomic sequencing data are available as a FASTQ file in the European Nucleotide Archive (ENA) of the European Bioinformatics Institute with the accession number PRJEB85279.

### Quantification of faecal short-chain fatty acids (SCFAs)

The quantification of SCFAs in the stool samples was carried out according to the procedure described below. Briefly, 100 mg of stools were suspended in 2 ml of 0.001% formic acid, stirred for 1 minute and centrifuged at 1000 g for 2 minutes at 4°C. The supernatant was recovered and the pellet was re-extracted. The supernatant was recovered and the pellet was again extracted following the same procedure. The two supernatants were then combined, and the volume was brought to 5 ml with a 0.001% formic acid solution. All the extracts were stored at - 20°C until the time of analysis. The analysis was performed with UPLC-HR-MS on an ACQUITY UPLC separation module (Waters, Milford, MA) coupled with an Exactive Orbitrap MS by means of a HESI-II probe for electrospray ionisation (Thermo Scientific, San Jose, CA). Elution was performed at a flow rate of 0.2 ml/min with the following solvents: 0.001% HCOOH in water treated with MilliQ (solvent A) and CH₃OH (1:1 v/v, solvent B). The elution gradient was the following: 0% B for 4 minutes, 0-15% B for 6 minutes, 15-20% B for 5 minutes, 20% B for 13 minutes, followed by a return to the initial conditions in 1 minute. The UPLC eluate was analysed in the MS full scan mode in the 50-130 m/z range. The external calibration curves were created using Sigma-Aldrich reagents (Milan, Italy) to quantify the acetic, butyric, isobutyric, isovaleric, propionic and valeric acids in the stool samples. The SCFA concentrations were expressed in mmol per kilogram of wet stools.

### Statistical analysis of microbiome

R programming language version 4.3.1 was used to exhaustively compare the GM and SCFAs between the active and placebo groups at the different timepoints V2, V4 and V5.

Alpha diversity measures the richness and evenness within a sample.

QIIME2 was used to calculate four indices: observed characteristics (richness), Shannon entropy (richness and evenness), Pielou's index (evenness) and Faith's phylogenetic diversity (richness and evolutionary diversity). Beta diversity, which evaluates between-group differences, was analysed using ANOSIM on the weighted and unweighted UniFrac distances to incorporate the phylogenetic relationships.

Different statistical methods were used to analyse the microbiome data. PLSDA and sPLSDA were applied to CLR transformed taxonomic data to distinguish between the active and placebo treatment groups, with ROC curves and matrices used to validate the model performances. The Shapiro-Francia test was used to assess data normality, followed by the Wilcoxon signed-rank test or paired-sample T-test and the Mann-Whitney test or T-test for unpaired data. The SCFA levels were compared among the various timepoints. LEfSe analysis identified the microbial biomarkers and the changes in SCFAs between the groups over time. The Kendall correlation was used to assess the relationships between gut bacteria, clinical parameters, SCFAs and alpha diversity. Random Forest analysis further validated the results by building multiple decision trees to improve prediction accuracy. It was carried out using the R "random Forest" library (available at http://www.stat.berkeley.edu/users/breiman/).

### Prediction of GM functional pathways

On the basis of the KEGG Orthology (KO) database, Phylogenetic Investigation of Communities by Reconstruction of Unobserved States of Correlation 2 (PICRUSt2) software was used to predict the functional pathways.

In order to identify different statistically significant (p-value ≤ 0.05) KO pathways between the active and placebo groups, the Mann-Whitney test was applied at each time-point V2, V4, and V5. Furthermore, to determine the direction of each KO pathway, the ratio between the mean abundance of the active and placebo groups was calculated, considering only the "unique" KOs associated with each timepoint.

### Clinical outcomes

From the screening visit and for the whole duration of the study, patients recorded data regarding their daily bowel habits (frequency and consistency of stools, rated with the Bristol Stool Form Scale) and weekly abdominal pain, bloating and general well-being, rated with a weekly numerical scale from "0" (none) to "10" (very severe) in a paper diary. In order to assess the patient-reported outcomes (PROs), patients were asked to respond to a "yes" or "no" question about their perception of the treatment's effectiveness. In particular, the following question: "Do you feel that this treatment has significantly improved your IBS symptoms?" was asked during the 3rd (phone call) and 4th checks of the study. Furthermore, patients were asked to fill out an IBS-SSS questionnaire on a weekly basis.

### Quality of life

At baseline, at the end of the treatment period and at the end of the follow-up, the quality of life was assessed by means of the 36-Item Short Form Health Survey (SF-36).

### 2. Results

42 patients were enrolled in the study and 35 were randomised (28 were included in the per-protocol population, PP). Table 4 describes the characteristics of the enrolled patients.

Figure 2 shows the flow diagram of participating patients.

The treatment was generally well tolerated. 5 patients (3 in the placebo group and 2 in the active group) manifested at least one side effect (AE) (15%, CI95% 4.95%-31.1%) for a total of 6 AEs. No severe AEs were recorded. The AEs reported were: headache, COVID-19, and flu-like syndrome in the placebo group and suspected oxyuriasis and upper abdominal pain (twice in the same patient) in the active group.

**Table 4: Demographic characteristics (ITT)**

| | | Statistical summaries | Total (n=31) | Placebo (n=15) | Active (n=16) | p-value |
|---|---|---|---|---|---|---|
| SEX | F | %, n | 54.8% (17/31) | 53.3% (8/15) | 56.3% (9/16) | 0.870 |
| | M | %, n | 45.2% (14/31) | 46.7% (7/15) | 43.8% (7/16) | |
| | | | | | | |
| Age | | Mean (SD) | 36.26 (10.652) | 37.40 (10.439) | 35.19 (11.077) | 0.526 |
| | | | | | | |
| WEIGHT (kg) | | Mean (SD) | 68.14 (11.074) | 68.26 (10.676) | 38.03 (11.784) | 0.984 |
| | | | | | | |
| HEIGHT (cm) | | Mean (SD) | 169.73 (7.625) | 167.64 (5.852) | 171.56 (8.664) | 0.307 |
| | | | | | | |
| ROME IV | IBS-D | %, n | 71.0 (22/31) | 73.3 (11/15) | 38.8 (11/16) | 0.999 |
| | IBS-M | %, n | 29.0 (9/31) | 26.7 (4/15) | 31.3 (5/16) | |
| | | | | | | |
| Disease duration (months) | | Mean (SD) | 538.19 (649.237) | 592.27 (681.304) | 487.50 (635.706) | 0.566 |
| IBS-SSS | | Mean (SD) | 243.40 (88.10) | 250.08 (78.42) | 237.17 (98.63) | 0.701 |
| Mild (<174) | | %, n | 29.03 (9/31) | 9.68 (3/31) | 19.35 (6/31) | |
| Moderate (175-300) | | %, n | 32.26 (10/31) | 19.35 (6/31) | 12.90 (4/31) | |
| Severe (>300) | | %, n | 32.26 (10/31) | 16.13 (5/31) | 16.13 (5/31) | |
| Missing | | %, n | 6.45 (2/31) | 3.23 (1/31) | 3.23 (1/31) | |
| Stool consistency (Bristol scale) | | Media (SD) | 4.48 (1.25) | 4.14 (1.44) | 4.80 (1.01) | 0.182 |
| 1-2 | | %, n | 1 | 1 | 0 | |
| 3-4-5 | | %, n | 18 | 10 | 8 | |
| 6-7 | | %, n | 8 | 2 | 6 | |
| Missing | | %, n | 4 | 2 | 2 | |

### Intestinal barrier dysfunction and intestinal permeability

The LaMa results at baseline were available for 31 patients. There was no significant difference between the two groups, in the ITT group or in the PP group, in the mean values of the LaMa ratio at baseline: the placebo group and the active group showed a mean LaMa ratio of 0.08 (SD 0.074) vs 0.05 (SD 0.035) in the ITT population and 0.08 (SD 0.077) vs 0.05 (SD 0.035) in the PP population, respectively. From baseline to week 12, there were no significant between-group or within-group changes in the mean LaMa ratio, either in the ITT population or in the PP one. In fact, at week 12 the mean LaMa ratio in the placebo and active groups was 0.08 (SD 0.068) vs 0.05 (SD 0.028) for ITT and 0.08 (SD 0.070) vs 0.05 (SD 0.028) for PP. Only 55% (n=17/31) of the patients showed an increase in the baseline LaMa ratio, without significant differences between the distribution in the placebo group and in the active group (Figure 3). However, after the treatment, a significant increase was observed in the percentage of patients with high permeability in the placebo group, but not in the active group.

A tendency towards normalisation of the LaMa ratio was observed in the active group.

In fact, in the ITT population, in the active group 25% of the patients showed a normalisation of intestinal permeability, 56.3% showed no changes compared to baseline and 18.8% showed an increase in intestinal permeability after the treatment, notwithstanding normal baseline values. In the placebo group, only 6.7% of the patients showed normalisation of an increase in intestinal permeability, 66.7% showed no changes and 26.7% worsened after the treatment (normal permeability at baseline and increase in permeability at week 12).

Only 12 patients out of 31 showed increased zonulin values (>48 ng/ml) at baseline. The mean zonulin values showed a progressive reduction in the active group, whilst stable values were observed in the placebo group (Figure 4).

In the ITT population, the patients receiving the placebo showed a mean change of -4.25 at week 12 and -0.47 (p=ns) at week 16. In the active group, by contrast, the patients reached a mean change of -5.86 (p=ns) at week 12 and -19.2 at week 16 (p<0.0001, with reference to the within-group change compared to baseline). At week 16 the mean difference in change compared to baseline was significantly higher in the active group compared to the placebo group (p=0.0046). In the PP population, the patients in the active group showed a reduction in zonulin of - 10.2 at week 12 compared to baseline (p=0.0375) versus -4.25 in the placebo group (p=ns). At week 16, the zonulin continued to decrease in the active group (-19.5, p=0.0002 compared to baseline), whereas it tended to return to baseline values in the placebo group (-0.47, p=ns compared to baseline). The mean difference in change between the two groups reached significance at week 16 (-19.01, p =0.0053).

### Vitamin D

The mean vitamin D levels were similar in the active and placebo groups at baseline (ITT 29.51 ± 11.08 vs 22.85 ± 7.03; PP 28.85 ± 11.10 vs 22.85 ± 7.03 in the active group and in the placebo group, respectively) and increased significantly in the active group after 12 weeks of treatment (ITT 43.88 ± 13.47 vs 25.73 ± 8.62, p=0.0021; PP 43.13 ± 14.57 vs 25.73 ± 8.62, p=0.0054 for the active group and placebo group, respectively).

### Clinical outcomes

Evaluating the percentage of days of abnormal bowel movements, where a day with normal bowel movements is defined as a day with less than or equal to 3 bowel movements a day and a Bristol Stool Form Scale value less than or equal to 5, in the PP population (Figure 5), the patients in the placebo group showed a trend towards improvement during the treatment but without significant results and an increase in the abnormal days at week 16, numerically higher compared to baseline. The patients in the active group showed a more stable trend towards improvement and also maintained this trend after interrupting the treatment, with a significant improvement compared to baseline at week 15 (-23. 2, p=0.0265) and at week 16 (-31.6, p=0.0049).

### Quality of life

The change in the social functioning score was significantly higher in the active group than in the placebo group (in the ITT group but not in the PP group) at week 16, with a mean increase of 20 (5.99) points in the active group and 0.83 (5.99) in the placebo group (p=0.028) (Table 5)

**Table 5: Change in social functioning score**

| Placebo | | | | | | Active | | | | | |
|---|---|---|---|---|---|---|---|---|---|---|---|
| | | | | change relative to the reference value | | | | change relative to the reference value | | Active vs Placebo | |
| Parameter | Visit | n | mean (SE) | mean (SE) | p-value | n | mean (SE) | mean (SE) | p-value | Mean diff in change Active-Placebo (95% CI) | p-value |
| SOCIAL FUNCTIONING | 0 | 15 | 60.39 (4.65) | | | 15 | 57.11 (4.65) | | | | |
| | 12 | 15 | 64.55 (4.65) | 4.17 (5.99) | 0.4898 | 16 | 69.61 (4.65) | 12.50 (5.99) | 0.0416 | 8.33(-8.65; 25.31) | 0.329 8 |
| | 16 | 15 | 61.22 (4.65) | 0.83 (5.99) | 0.8899 | 16 | 77.11 (4.65) | 20.00 (5.99) | 0.0015 | 19.17(2.19; 36.15) | 0.027 7 |

### Ecological and functional GM analysis

The GM analyses were conducted on 27 patients (PP set) included in this analysis (15 in the placebo group and 12 in the active probiotic group). Alpha and beta diversity analyses were conducted.

A paired statistical test was performed to compare the modulation of the gut microbiome (GM) in the active and placebo groups at all timepoints (V2, V4, V5). In particular, a modulation of various bacteria was observed after the administration of the active IP treatment vs the placebo. In particular, an increase in two specific bacteria was recorded in the group receiving the active formulation: *Bifidobacterium animalis* subsp. *lactis* and *Streptococcus thermophilus.* Furthermore, different species of the family Lachnospiraceae showed to be more abundant after the active treatment vs the placebo group. However, *Lachnospira* showed a decrease in the subjects treated with the active formulation as opposed to the placebo. Furthermore, a single species of the genus *Dorea* increased in the group receiving the active product, whilst the genus D*o*rea showed a significant increase after administration of the placebo. It is interesting to note that a species belonging to Ruminococcaceae increased after the active treatment, showing an opposite trend compared to the placebo.

As regards SCFAs, an increase in lactic acid was observed after the active treatment and treatment with the placebo, and in acetate only after treatment with the placebo (Figure 2).

Using LEfSE the approximately comparable results were confirmed for the active treatment. However, an increase in *Enterococcus* and a decrease in *Actinomyces* was observed in the placebo group (Figure 6). With regard to SCFAs, lactate increased in both treatments, whereas succinate increased after administration of the active IP and acetate after administration of the placebo.

Comparing the successive timepoints V4 and V5, no significant changes were detected with the placebo treatment. However, after the active treatment a decrease in *Bifidobacterium animalis* subsp. *lactis* and in the family Lactobacillaceae was recorded, in addition to an increase in a species of the genus *Ruminococcus.*

### GM comparison between subjects after treatment

At the timepoint V4, at week 12, the subjects were compared in an unpaired manner using the Mann-Whitney test to observe the differences deriving from the different treatments. The active group showed a significant increase in Actinobacteria and a decrease in Proteobacteria. In order to explore these results further, sPLS-DA was performed to determine whether the subjects could be grouped based on the treatment received and whether machine learning techniques could predict the treatment a subject had undergone (Figure 7). The most important variables for distinguishing the two groups were *Bifidobacterium animalis* subsp. *lactis,* the alpha diversity index Faith's PD (which is based on the richness and phylogenetic relationships of bacteria) and the SCFAs, in particular acetate and butyrate.

sPLS-DA was also performed on two groups: V4 group after the active treatment and V4 group after treatment with the placebo, together with a third group from the timepoint V2, which included all the subjects before any treatment (Figure 8). The classification suggested that the subjects in the V4 placebo group were more similar to subjects at point V2 than to those in the V4 active treatment (IP) group. These results were confirmed by another machine learning algorithm, random forest, which showed an accuracy of 0.69 in distinguishing between the V2 group and the V4 placebo group. In contrast, the comparison between the V4 active and V4 placebo groups produced a classification accuracy of 0.8, with *Bifidobacterium animalis* subsp. *lactis, Streptococcus thermophilus, Lachnospira,* Lactobacillaceae and another undefined species of *Bifidobacterium* emerging as the most important discriminant variables. These results are consistent with those of the Mann-Whitney test used to compare the two groups.

### Correlation between the potential microbial biomarkers after the active treatment and clinical parameters, SCFAs and alpha diversity indices

Correlation analysis reveals the complex interactions between the gut microbiome, clinical parameters, SCFAs and alpha diversity indices, highlighting the potential of gut microbiota modulation in the treatment of irritable bowel syndrome. Starting from the bacteria administered with the active compound, *Bifidobacterium animalis* subsp. *lactis* was negatively correlated with butyrate, whereas *Streptococcus thermophilus* showed a negative correlation with citrulline.

As regards the bacteria modulated by the active compound, a species of the family Lachnospiraceae was positively correlated with physiological function in the subjects. *Lachnospira,* which decreased after the treatment, was correlated positively with zonulin and negatively with the alpha diversity evenness index. Furthermore, *Ruminococcus* was positively correlated with the SF-36 pain domain, where low levels indicate severe abdominal pain and high levels indicate absence of pain. Furthermore, an unidentified species of the genus Dorea increased after the treatment and was positively correlated with the alpha diversity evenness index.

### Prediction of functional pathways based on PICRUSt.

KO pathways (p-value<0.05), overexpressed in the active group as compared to the placebo group, were revealed at times V2, V4 and V5. In particular, at V2 the following KO pathways were identified: biosynthesis of unsaturated fatty acids; glycolysis/gluconeogenesis (K01792); messenger RNA biogenesis; nicotinate and nicotinamide metabolism; peptidase and inhibitors; propanoate metabolism (K123788); and bicomponent system.

At V4, the KOs identified were: arabinogalactan biosynthesis - mycobacterium (K02851); biosynthesis of unsaturated fatty acids; cysteine and methionine metabolism (K08969); messenger RNA biogenesis (K08300); peptidoglycan biosynthesis (K00887-K05363); phenylalanine, tyrosine and tryptophan biosynthesis (K04092); terpenoid backbone biosynthesis (K13787); and thiamine metabolism (K14153). Finally, at V5, the KOs identified were: aminoacyl-tRNA biosynthesis (K02433-K02434-K02435); arginine biosynthesis (K00620); bacterial motility proteins (K02650-K02662); glycerophospholipid metabolism (K01048); histidine metabolism (K01496-K01523-K01693); novobiocin biosynthesis (K04517); peptidoglycan biosynthesis (K05364-K07009); peptidoglycan degradation and biosynthesis proteins (K07284); ribosome biogenesis (K06442); starch and sucrose metabolism (K00702); and transcription factors (K03705).

### 3. Conclusions

Irritable bowel syndrome is a common intestinal disorder with a pathogenesis that is still unclear. However, there is increasingly strong evidence to suggest a role of a dysregulated gut-brain axis with dysbiosis and alteration of the intestinal barrier, which play an important role in determining abdominal pain and disturbed motility. In recent years, the criteria for the diagnosis of IBS have been revised, with a focus on the presence of pain as a key symptom and the discarding of more nonspecific symptoms such as abdominal discomfort to define a diagnosis of IBS. However, the diagnostic criteria are based solely on symptoms and do not include any objective measure or biomarker. Therefore, even when the most up-to-date Rome IV criteria are applied, there exists the possibility of including in the same clinical diagnosis patients affected by IBS who show a different intestinal barrier function and a state of the gut microbiota in which intestinal barrier impairment could play a different role in determining symptoms.

It is interesting to note that the patients treated with the mixture according to the invention showed a progressive trend towards improvement in the percentage of days characterised by abnormal bowel movements, which became significant 4 weeks after interruption of the treatment with probiotics + vitamin D when compared to the placebo group, and a better stool consistency 4 weeks after interruption of the treatment.

Advantageously, a corresponding progressive decrease in serum zonulin was observed during the whole period of observation in the active group; it reached statistical significance compared to baseline at week 12 and at week 16 and compared to the placebo 4 weeks after interruption of the treatment, meaning a progressive restoration of tight junctions and of the epithelial barrier.

Furthermore, these results suggest that the mixture according to the invention acts mainly on the integrity of the epithelial barrier, as demonstrated by the significant reduction in zonulin, without influencing the enterocyte mass. Using the LaMa ratio as a marker of intestinal permeability, a trend towards fluctuating results at different moments was found, suggesting that intestinal permeability is a variable measure, which is reactive to the environment and could be unstable in IBS. However, the combined supplementation with the probiotics according to the mixture of the invention and tested in the study and vitamin D showed a stabilising effect on intestinal permeability, since the active group showed similar percentages of patients with increased and normal permeability compared to baseline (50% of patients with normal permeability at week 0 vs 56.3% at week 12), whereas in the placebo group a larger percentage of patients showed an increase in permeability at week 12, even though the percentages were similar at baseline (40% of patients with normal permeability at baseline vs 20% at week 12).

It is interesting to note that in the study cohort the mixture according to the invention seemed to take a long time to act and, surprisingly, continue to act also after interruption of the treatment. This is explained by the effect that probiotics have on the host microbial species. We know that probiotics are capable of colonising the intestine while they are being taken and disappear rapidly after interruption of the treatment, without a relevant impact on the composition of the host microbiome.

The analysis of alpha diversity, which measures within-sample diversity, in the study cohort did not reveal any significant differences between the timepoints in either of the treatment groups. Similarly, the analysis of beta diversity using UniFrac did not show any grouping of subjects, indicating a stable microbial diversity both within and between groups over time.

Furthermore, a modulation of specific bacterial taxa was observed between the active and placebo groups. In the active group, as predicted, there was a recorded increase in *Bifidobacterium animalis* subsp. *lactis* and *Streptococcus thermophilus,* both included in the mixture according to the invention.

In particular, *Bifidobacterium* was present only during the treatment period, indicating that it does not persist in the subject's microbiome after the end of treatment.

In contrast, *Streptococcus* was still detectable, suggesting that *Streptococcus* shows a greater persistence than *Bifidobacterium,* even though it is less common to find *Streptococcus* in such a persistent state.

In the study cohort, various species of the family *Lachnospiraceae* increased after the treatment, with the exception of the genus *Lachnospira,* which decreased. The family *Lachnospiraceae* plays a crucial role in SCFA production.

Furthermore, it was found that *Lachnospira* is correlated with a decrease in zonulin parameters after an active treatment.

It is interesting to note that the genus *Dorea* showed a species-specific response: one species increased in the probiotics group, whereas the genus overall increased in the placebo group. The role of *Dorea* in the gut is controversial, because it has been associated with an increase in gas production and intestinal permeability, factors believed to contribute to the physiopathology of irritable bowel syndrome. *Dorea* is involved in the fermentation of complex carbohydrates, which may lead to the production of gas and SCFAs. An excess of SCFAs can have both positive and negative effects on gut health in patients with IBS without constipation. In particular, a positive correlation was observed between *Dorea* and the alpha diversity evenness index. A healthy gut typically shows a high alpha diversity, associated with greater resilience and functionality and better health outcomes.

Though *Ruminococcaceae,* known for fibre degradation and SCFA production, also increased in the active group, no corresponding increase in SCFA production was observed. It was possible to observe a positive correlation between this family and the pain index, suggesting a potential role in alleviating pain. The levels of lactic acid increased in both groups, whereas those of acetate increased only in the placebo group. LEfSE analysis confirmed the observed modulation models, indicating minimal changes in the SCFA levels between the placebo group and the active one, but nonetheless showing a bacterial modulation in the active group.

As regards the differences observed between the samples after treatment, the machine learning results suggest a more substantial change in the microbiota after the active treatment as compared to the placebo. Considerable changes in the amounts of *Proteobacteria* and *Actinobacteria* emerged. In particular, *Proteobacteria* decreased and *Actinobacteria* increased in the active group. These data suggest a positive effect of the active treatment. Actinobacteria, in particular the genus *Bifidobacterium,* are often reduced in patients affected by IBS.

The correlation analysis revealed complex interactions between the gut microbiome, clinical parameters, SCFAs and alpha diversity indexes, suggesting that microbiome modulation may have the potential of improving the symptoms of IBS without constipation.

Whilst the overall microbial diversity remained stable, specific bacteria were significantly influenced by the supplementation with the mixture according to the invention.

In particular, the PICRUSt prediction showed a passage to metabolism of SCFA producers in V5, with particular reference to the "starch and sucrose metabolism" KO, which suggests an association with *Ruminococcaceae, Erysipelotricaceae* and other SCFA-producing bacteria.

Furthermore, the novobiocin biosynthesis KO was detected only in V5, suggesting the biosynthesis of antimicrobial peptides only after the end of the treatment, with a shift in the ecological and functional situation between the interruption of administration (V4) and the 4 weeks of follow-up (V5).

In the study cohort, in the first place, the probiotics were capable of colonising the gut, since species correlated to the probiotics were already detected after 12 weeks of therapy. However, after 12 weeks of therapy the probiotic species probably had not yet begun to have a significant impact on the host microbiota or on the intestinal barrier. These interactions take time to develop and could become increasingly significant with the passing of time, even after the interruption of oral supplementation with the mixture according to the invention, since at 16 weeks, after a 4-week washout, the probiotic species had disappeared, and a different microbial composition emerged as compared to baseline.

It is probably at week 16 that the changes in the gut microbiota began having a positive and significant impact on the entire barrier function, as demonstrated by the further improvement in intestinal barrier function based on the zonulin levels at week 16.

Without wishing to be bound by any theory, vitamin D supplementation could act simultaneously on the deeper layers of the intestinal barrier through the activation of vitamin D receptors on epithelial and immune cells. In fact, vitamin D has demonstrated a pleiotropic effect on the regulation of the integrity of intestinal epithelial cells, the induction of immunotolerance and, in general, the regulation of the immune system, with a consequent overall improvement in intestinal barrier function and permeability.

In this scenario, it is not surprising that the clinical improvement followed the timing of the improvement in the intestinal barrier. The beneficial effect on the intestinal barrier probably temporarily precedes the clinical effects and we may hypothesise that a hypothetical longer follow-up, beyond the 16th week, would give the possibility of finding a more profound clinical effect tied to a lightening of the intestinal barrier.

## Claims

1. A mixture comprising or, alternatively, consisting of:
i) a bacterial strain belonging to the species *Bifidobacterium animalis* subsp. *lactis,* wherein said strain is the bacterial strain *Bifidobacterium animalis* subsp. *lactis* BL03 deposited by Beingpharma S.r.l. at the BCCM/LMG/LMG on 06.03.2025 with the deposit number **LMG P-34006** and a bacterial strain belonging to the species *Bifidobacterium animalis* subsp. *lactis,* wherein said strain is the bacterial strain *Bifidobacterium animalis* subsp. *lactis* BI04 deposited by Beingpharma S.r.l. at the BCCM/LMG/LMG on 06.03.2025 with the deposit number **LMG P-34007;** and
ii) at least one bacterial strain belonging to the species *Streptococcus thermophilus,* wherein said strain is the bacterial strain *Streptococcus thermophilus* BT01 deposited by Beingpharma S.r.l. at the BCCM/LMG/LMG on 06.03.2025 with the deposit number **LMG P-34002;** and
iii) at least one bacterial strain belonging to the species *Lactiplantibacillus plantarum,* wherein said strain is the bacterial strain *Lactiplantibacillus plantarum* BP06 deposited by Beingpharma S.r.l. at the BCCM/LMG/LMG on 06.03.2025 with the deposit number **LMG P-34005;** and
iv) at least one bacterial strain belonging to the species *Lactobacillus acidophilus,* wherein said strain is the bacterial strain *Lactobacillus acidophilus* BA05 deposited by Beingpharma S.r.l. at the BCCM/LMG/LMG on 06.03.2025 with the deposit number **LMG P-34008;** and
v) at least one bacterial strain belonging to the species *Lacticaseibacillus paracasei,* wherein said strain is the bacterial strain *Lacticaseibacillus paracasei* BP07 deposited by Beingpharma S.r.l. at the BCCM/LMG/LMG on 06.03.2025 with the deposit number **LMG P-34004;** and
vi) at least one bacterial strain belonging to the species *Lactobacillus helveticus,* wherein said strain is the bacterial strain *Lactobacillus helveticus* BD08 deposited by Beingpharma S.r.l. at the BCCM/LMG/LMG on 06.03.2025 with the deposit number **LMG P-34003;** and
vii) at least one bacterial strain belonging to the species *Bifidobacterium breve,* wherein said strain is the bacterial strain *Bifidobacterium breve* BB02 deposited by Beingpharma S.r.l. at the BCCM/LMG/LMG on 06.03.2025 with the deposit number **LMG P-34009;** or mixtures thereof; and
viii) vitamin D.

2. The mixture according to any one of the preceding claims, wherein one or more of said bacterial strains i) - vii) are in viable form, in viable but non-replicating form, or in non-viable, inactivated, semi-inactivated, or dead form; said bacterial strains i) - vii) are preferably in viable form or in viable but non-replicating form.

3. The mixture according to any one of the preceding claims, wherein one or more of said bacterial strains i) - vii) are each in a concentration comprised from 1x10⁵ CFU/g to 1x10¹³ CFU/g, more preferably from 1x10⁶ CFU/g to 1x10¹² CFU/g.

4. The mixture according to any one of the preceding claims, wherein said viii) vitamin D in said mixture is preferably vitamin D3.

5. A composition comprising a mixture according to any one of claims 1-4 and, optionally, excipients and/or additives of acceptable food grade or pharmaceutical grade.

6. The composition according to claim 5, wherein said mixture is present in said composition in an amount by weight comprised from 10% to 90%, preferably from 25% to 75%, more preferably from 40% to 60%, whilst said at least one conventional carrier and/or excipient are present in an amount by weight comprised from 90% to 10%, preferably from 75% to 25%, more preferably from 60% to 40% by weight, relative to the total weight of the composition.

7. At least one bacterial strain selected in the group comprising or, alternatively, consisting of:
*i) Bifidobacterium animalis* subsp. *lactis BL03* **(LMG P-34006)** and *Bifidobacterium animalis* subsp. *lactis* BI04 **(LMG P-34007);**
ii) *Streptococcus thermophilus* BT01 **(LMG P-34002);**
iii) *Lactiplantibacillus plantarum BP06* **(LMG P-34005);**
iv) *Lactobacillus acidophilus* BA05 **(LMG P-34008);**
*v) Lacticaseibacillus paracasei BP07* **(LMG P-34004);**
vi) *Lactobacillus helveticus BD08* **(LMG P-34003);**
vii) *Bifidobacterium breve BB02* **(LMG P-34009),** or mixtures thereof
for use in a method of treatment, preventive or curative, of irritable bowel syndrome and/or disorders associated therewith; more preferably for use in the treatment of irritable bowel syndrome not **characterised by** constipation in subjects with a diagnosis according to Rome IV criteria.

8. The at least one bacterial strain for use according to claim 7, wherein said single bacterial strain is in combination with a vitamin D; preferably a vitamin D3.

9. The at least one bacterial strain according to claim 7 or 8, for use in a method for reducing intestinal permeability in subjects with a diagnosis of irritable bowel syndrome without constipation, wherein said diagnosis is made according to Rome IV criteria.

10. The mixture according to any one of claims 1-4, for use as a medicament or a food.

11. The composition according to claim 5 or 6, for use as a medicament or a food.

12. The mixture or composition for use according to claim 10 or 11, wherein said mixture and/or said composition are preferably for use in the reduction of intestinal dysbiosis and/or for use in a method of treatment, preventive or curative, of irritable bowel syndrome and/or disorders associated therewith; preferably for use in the treatment of irritable bowel syndrome and/or disorders associated therewith, wherein said diagnosis is made according to Rome IV criteria.

13. The mixture or composition for use according to any one of claims 10-12, for use in a method for reducing intestinal permeability in subjects with a diagnosis of irritable bowel syndrome without constipation, wherein said diagnosis is made according to Rome IV criteria.

14. The composition according to any one of claims 10-13, for oral use, preferably in the form of a capsule, a tablet, a sachet, a stick, an orodispersible stick, a gel for oral use or granules.
